(19) Europäisches Patentamt
European Patent Office
Office européen des brevets

(11) Veröffentlichungsnummer: **0 161 219 B1**

(12) **EUROPÄISCHE PATENTSCHRIFT**

(45) Veröffentlichungstag der Patentschrift: **12.06.91**

(51) Int. Cl.5: **C09D 5/08**

(21) Anmeldenummer: **85810209.8**

(22) Anmeldetag: **06.05.85**

Die Akte enthält technische Angaben, die nach dem Eingang der Anmeldung eingereicht wurden und die nicht in dieser Patentschrift enthalten sind.

# (54) Heterocyclische Mercaptocarbonsäure-ester und -anhydride als Korrosionsinhibitoren.

(30) Priorität: **11.05.84 GB 8412063**

(43) Veröffentlichungstag der Anmeldung:
**13.11.85 Patentblatt 85/46**

(45) Bekanntmachung des Hinweises auf die Patenterteilung:
**12.06.91 Patentblatt 91/24**

(84) Benannte Vertragsstaaten:
**AT BE CH DE FR GB IT LI NL SE**

(56) Entgegenhaltungen:
**EP-A- 0 003 817**
**CH-A- 458 048**

(73) Patentinhaber: **CIBA-GEIGY AG**
**Klybeckstrasse 141**
**CH-4002 Basel(CH)**

(72) Erfinder: **Bentley, Robert Leonard**
**34 Eddisbury Avenue Urmston**
**Manchester M31 2QJ(GB)**
Erfinder: **Hoyle, William**
**29 Carrwood Avenue Bramhall**
**Stockport Cheshire SK7 2PY(GB)**
Erfinder: **Jack, James**
**6, Huxley Drive Bramhall**
**Stockport Cheshire SK7 2PH(GB)**

EP 0 161 219 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft die Verwendung von heterocyclischen Mercaptocarbonsäure-estern und -anhydriden als Korrosionsinhibitoren, die mittels dieser Verbindungen korrosionsgeschützten Systeme und die Verbindungen selbst, soweit sie neu sind.

Wirkungsvoller Korrosionsschutz ist eine der wichtigsten Erfordernisse für organische Anstrichstoffe auf Metallsubstraten. Viele Vorschläge zur Verbesserung der antikorrosiven Wirkung von Anstrichen wurden bereits gemacht, siehe H. Kittel, Lehrbuch der Lacke und Beschichtungen, Band V, (1977), 46-103. Ein Weg hierzu ist die Verbesserung der Barrierewirkung des Anstriches um den Zutritt von Sauerstoff, Wasser und Jonen zur Metalloberfläche zu unterbinden. Ein anderer Weg ist der heutzutage übliche Zusatz von antikorrosiven Pigmenten, welche chemisch oder elektrochemisch in den Korrosionsprozess eingreifen durch Bildung unlöslicher Abscheidungen mit den primären Korrosionsprodukten wie Alkali- oder Metallionen oder durch Passivierung der Metalloberfläche. Zu den wirksamsten antikorrosiven Pigmenten gehören Metallchromate und Bleiverbindungen (z.B. Oxide). Metallchromate wurden vielfach als antikorrosive Pigmente benutzt, weil sie sowohl die anodische wie die kathodische Korrosion inhibieren können. Neuerdings bestehen jedoch ökologische Bedenken gegen die Verwendung von Chromaten wegen ihrer potentiellen Karzinogenität. In ähnlicher Weise bestehen grosse Bedenken gegen die Verwendung von Bleiverbindungen in Anstrichen wegen deren Toxizität.

Metallsalze von organischen Verbindungen sind ebenfalls als Korrosionsinhibitoren für Anstriche vorgeschlagen worden. So wird z.B. in der Europ. Patentschrift 3817 die Verwendung von Zink- oder Bleisalzen von Hydroxyl- oder Sulfhydrylverbindungen von 5- oder 6-gliedrigen Heterocyclen, die die charakteristische Gruppe -N=C(OH)- oder -N=C(SH)- enthalten, beschrieben. Typissche Beispiele dafür sind die Zink- und Bleisalze von 2-Mercaptobenzthiazol. Gegen die Verwendung solcher Bleisalze bestehen toxikologische Bedenken.

Wie in Farbe und Lack 87, 787 (1981) dargelegt wurde, bestehen in der Fachwelt Zweifel, ob der Zusatz organischer Korrosionsinhibitoren allein (ohne Korrosionsschutz-Pigmente) einen für die Praxis ausreichenden Korrosionssschutz auf dem Anstrichsektor gewährleisten kann.

Aehnliche Ueberlegungen gelten für wässrige Systeme, die mit Metallen in Kontakt stehen wie z.B. Kühl- oder Heizwasserkreisläufe, hydraulische Flüssigkeiten oder Metallbearbeitungsflüssigkeiten. Auch hier kann die Korrosion des Metalls durch Zusatz von Korrosionsinhibitoren zur wässrigen Flüssigkeit vermindert werden, es bestehen jedoch erhebliche ökologische Bedenken wegen der potentiellen Vergiftung des Abwassers.

Es wurde nunmehr gefunden, dass bestimmte heterocyclische Carbonsäure-ester und -anhydride und nicht-toxische Salze solcher Verbindungen wirkungsvolle Korrosionsinhibitoren sind und sowohl in Anstrichstoffen wie in wässrigen Systemen als solche verwendet werden können. Ihr Vorteil ist eine niedrige Toxizität bei hoher Wirksamkeit.

Bei der Verwendung in Anstrichstoffen besteht gegenüber korrosionsinhibitierenden Pigmenten der Vorteil, dass man frei in der Wahl des Farbpigmentes ist und auch unpigmentierte Klarlacke antikorrosiv formulieren kann.

Die vorliegende Erfindung betrifft die Verwendung von Estern oder Anhydriden aliphatischer oder cycloaliphatischer Mono-, Di-, Tri- oder Tetracarbonsäuren, welche im aliphatischen oder cycloaliphatischen Rest durch eine oder mehrere, vorzugsweise durch eine Gruppe der Formel I substituiert sind,

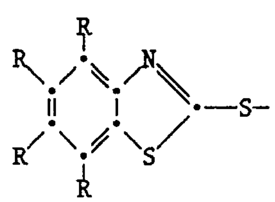

I

worin einer der Substituenten R Wasserstoff, $C_1$-$C_4$-Alkyl oder $C_1$-$C_4$-Alkoxy ist und die drei anderen R Wasserstoff sind, sowie von nicht-toxischen Salzen solcher Verbindungen als Korrosionsinhibitoren.

Die Erfindung betrifft ferner Anstrichstoffe oder wässrige Systeme ausserhalb der Anstrichstoffe, die eine wirksame Menge dieser Verbindungen als Korrosionsinhibitoren enthalten.

Unter "Ester" von aliphatischen oder cycloaliphatischen Mono-, Di-, Tri- oder Tetracarbonsäuren sind Voll- oder Teilester zu verstehen, die 1-4 Gruppen -COOZ enthalten. Z bedeutet $C_1$-$C_{18}$-Alkyl, das durch ein oder mehrere O- oder S-Atome oder $NR^\circ$-Gruppen unterbrochen sein kann ($R^\circ$ = $C_1$-$C_{18}$-Alkyl, $C_5$-$C_8$-

Cycloalkyl, Phenyl, Naphthyl, $C_7$-$C_9$-Phenylalkyl oder $C_7$-$C_{18}$-Alkylphenyl) oder durch -SH, $-COOR^{\circ}$, $-CONH_2$, -CN oder Halogen substituiert sein kann. Z bedeutet weiterhin $C_2$-$C_{10}$-Hydroxyalkyl (das durch eine oder mehrere $NR^{\circ}$-Gruppen oder O-Atome unterbrochen sein kann), $C_2$-$C_{18}$-Alkenyl, $C_3$-$C_{12}$-Cycloalkyl (das durch $C_1$-$C_4$-Alkyl, -OH, -SH, $-COOR^{\circ}$, $-CONH_2$, -CN oder Halogen substituiert sein kann), $C_7$-$C_9$-Phenylalkyl, $C_7$-$C_{18}$-Alkylphenyl, $C_6$-$C_{10}$-Aryl (das durch $C_1$-$C_{12}$-Alkoxy, $C_1$-$C_{12}$-Alkylthio, -COOH, -OH, $-NO_2$ oder Halogen substituiert sein kann). Wenn die Verbindung mehr als eine Gruppe -COOZ enthält, so können die Gruppen Z identisch oder verschieden sein. Teilester von Di-, Tri- oder Tetracarbonsäuren enthalten neben der Gruppe -COOZ noch mindestens eine Gruppe -COOH.

Unter Anhydriden von aliphatischen oder cycloaliphatischen Monocarbonsäuren sind symmetrische oder nicht-symmetrische Anhydride zu verstehen. Unter Anhydriden von aliphatischen oder cycloaliphatischen Di-, Tri- oder Tetracarbonsäuren sind cyclische Anhydride zu verstehen, die eine Gruppe der Formel III oder IIIa

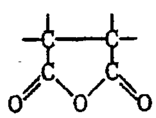 III

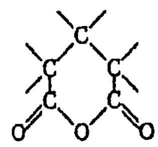 IIIa

enthalten.

Z als $C_1$-$C_{18}$-Alkyl kann gerade- oder verzweigtkettiges Alkyl sein, wie z.B. Methyl, Ethyl, n-Propyl, Isopropyl, n-Butyl, sec-Butyl, tert-Butyl, n-Pentyl, n-Hexyl, n-Heptyl, n-Octyl, 2-Ethylhexyl, tert-Octyl, n-Nonyl, n-Decyl, n-Dodecyl, n-Tetradecyl, n-Hexadecyl oder n-Octadecyl.

Z als durch O, S oder $NR^{\circ}$ unterbrochenes Alkyl kann z.B. Ethoxymethyl, 2-Methoxyethyl, 3-Ethoxypropyl, 2-Dodecyloxyethyl, 3,6-Dioxadecyl, 2-Butylthioethyl, 2-tert-Dodecylthioethyl, 2-(Dimethylamino)propyl, 2-(Dibutylamino)ethyl, 3-Butylaminopropyl oder 2-(Methyl-phenylamino)ethyl sein.

Z als durch SH, $COOR^{\circ}$, $CONH_2$, CN oder Halogen substituiertes Alkyl kann z.B. 2-Mercaptobutyl, 3-Mercaptopropyl, Ethoxycarbonylmethyl, 2-Isopropoxycarbonylethyl, 2-Carbamoylethyl, 2-Cyanoethyl, 2-Chlorethyl oder 3-Chlorpropyl sein.

Z als gegebenenfalls durch O oder $NR^{\circ}$ unterbrochenes $C_2$-$C_{10}$-Hydroxyalkyl kann z.B. 2-Hydroxyethyl, 2-Hydroxypropyl, 4-Hydroxybutyl, 6-Hydroxyhexyl, 5-Hydroxy-3-oxapentyl, 8-Hydroxy-3,6-dioxaoctyl, 11-Hydroxy-3,6,9-trioxaundecyl, 5-Hydroxy-3-(methylaza)-pentyl oder 9-Hydroxy-3,7-di(methylaza)-nonyl sein.

Wenn Z $C_2$-$C_{18}$-Alkenyl ist, so kann es unverzweigt oder verzweigtes Alkenyl sein, wie z.B. Vinyl, 2-Propenyl, Allyl, Methallyl oder Oleyl. Z als gegebenenfalls substituiertes $C_3$-$C_{12}$-Cycloalkyl kann z.B. Cyclopropyl, Cyclobutyl, Cyclopentyl, Cyclohexyl, Cyclooctyl, Cyclododecyl, Methylcyclohexyl, Dimethylcyclohexyl, tert.-Butylcylohexyl, 3-Hydroxycyclohexyl, 4-Mercaptocyclohexyl, 2-Ethoxycarbonylcyclohexyl, 4-Carbamoylcyclohexyl, 3,4-Dichlorcyclopentyl, 4-Fluorcyclohexyl oder 4-Cyanocyclohexyl sein.

Wenn Z $C_7$-$C_9$-Phenylalkyl ist, kann es z.B. Benzyl, 1-Phenylethyl, 2-Phenylethyl, $\alpha,\alpha$-Dimethylbenzyl oder 3-Phenylpropyl sein. Wenn Z $C_7$-$C_{18}$-Alkylphenyl ist, so kann es z.B. Tolyl, Xylyl, 4-Isopropylphenyl, 4-tert. Butylphenyl, 4-Octylphenyl oder 4-Dodecylphenyl sein.

Z als gegebenenfalls substituiertes Aryl kann z.B. Phenyl, Chlorphenyl, Dichlorphenyl, Nitrophenyl, Hydroxyphenyl, Methoxyphenyl, Isopropoxyphenyl, 4-(Methylthio)phenyl, 2-Carboxyphenyl, 1-Naphthyl, 2-Naphthyl oder 4-Chlor-1-naphthyl sein.

Bevorzugt ist Z $C_1$-$C_{18}$-Alkyl, $C_3$-$C_{12}$-Alkoxyalkyl, $C_2$-$C_6$-Hydroxyalkyl, Allyl, Cyclohexyl, Benzyl, Phenyl, Tolyl oder Naphthyl.

Bevorzugte Korrosionsinhibitoren sind solche der Formel II

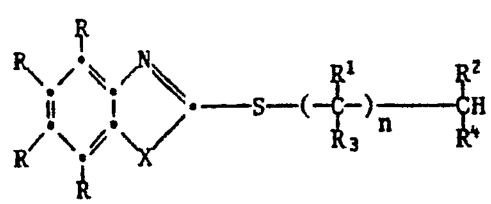 II

worin R die vorhin gegebene Bedeutung hat, n Null oder 1 ist, und $R^1$, $R^2$, $R^3$ und $R^4$, unabhängig

voneinander, Wasserstoff, $C_1$-$C_{18}$-Alkyl, $C_1$-$C_4$-Hydroxyalkyl, $C_1$-$C_4$-Halogenalkyl, $C_1$-$C_4$-Alkoxyalkyl, unsubstituiertes oder im Phenylkern durch Halogen oder Hydroxy substituiertes Phenyl oder Phenylalkyl, eine Gruppe -COOH oder -COOZ, worin Z die vorhin gegebene Bedeutung hat, oder durch 1, 2 oder 3 Gruppen -COOH oder -COOZ substituiertes Alkyl bedeuten oder worin im Rest

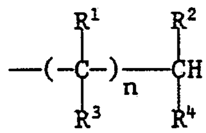

zwei Gruppen -COOH zu einer Anhydridgruppe der Formel III oder IIIa cyclisiert sind, sowie solche Verbindungen der Formel II, worin $R^1$ und $R^2$ oder $R^1$ und $R^3$ zusammen eine unverzweigte oder verzweigte Alkylengruppe bilden, die durch 1 oder 2 Gruppen -COOH oder -COOZ substituiert sein kann, sowie solche Verbindungen der Formel II, worin $R^1$ und $R^2$ zusammen eine direkte Bindung bilden, wobei in allen Fällen der Rest

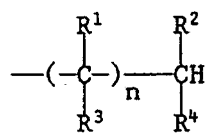

mindestens eine Gruppe -COOZ oder der Formel III oder IIIa enthält, sowie nicht-toxische Basenadditions-Salze solcher Verbindungen der Formel II, die eine oder mehrere Gruppen -COOH enthalten.

Bevorzugt sind Verbindungen der Formel II, worin n = 1 ist.

Die Anhydrid-Gruppen III oder IIIa können durch Cyclisierung von zwei Gruppen -COOH gebildet sein, beispielsweise von a) $R^1$, $R^2$, $R^3$ oder $R^4$ als -COOH b) $R^1$, $R^2$, $R^3$ oder $R^4$ als durch -COOH substituiertes Alkyl c) $R^1$ und $R^2$ zusammen oder $R^1$ und $R^3$ zusammen durch -COOH substituiertes Alkylen.

$R^1$, $R^2$, $R^3$ und $R^4$ als Alkyl sind vorzugsweise $C_1$-$C_{18}$-Alkyl, wie z.B. Methyl, Ethyl, n-Propyl, Isopropyl, n-Butyl, Isobutyl, tert. Butyl, n-Pentyl, n-Hexyl, n-Octyl, n-Dodecyl oder n-Octadecyl. Als Hydroxyalkyl oder Halogenalkyl haben diese Substituenten vorzugsweise 1-4 C-Atome, wie z.B. Hydroxymethyl, 1- oder 2-Hydroxyethyl, 2- oder 3-Hydroxypropyl, Chlormethyl, Bromethyl oder Bromisopropyl. Als Alkoxyalkyl haben diese Substituenten vorzugsweise 2-10 C-Atome, wie z.B. Methoxymethyl, 1-Methoxyethyl, 2-Ethoxypropyl, 1-Methoxyethyl, 2-Ethoxypropyl, 1-Methoxybutyl, n-Butoxymethyl oder 4-Isopropoxybutyl. $R^1$, $R^2$, $R^3$ und $R^4$ als durch 1, 2 oder 3 Gruppen -COOH oder -COOZ substituiertes Alkyl hat vorzugsweise 2-12 C-Atome. Z ist darin vorzugsweise $C_1$-$C_{18}$-Alkyl. Beispiele hierfür sind die Gruppen $-CH_2-COOCH_3$, $-CH_2CH_2COOH$, $-CH_2(CH_3)COOC_2H_5$, $-(CH_2)_3-COOC_2H_5$, $-(CH_2)_6-COOC_3H_7$-iso, $-CH_2-CH(COOH)-CH_2COOC_4H_9$, $-CH(COOH)-CH_2-COOCH_3$, $-CH_2(COOCH_3)-CH_2-CH_2COOCH_3$ oder $-CH_2-CH(COOC_2H_5)-CH(COOC_2H_5)-CH_2COOC_2H_5$.

Wenn $R^1$, $R^2$, $R^3$ und $R^4$ gegebenenfalls substituiertes Phenyl oder Phenylalkyl sind, so können sie z.B., 4-Chlorphenyl, Toly, Xylyl, 3-Methoxyphenyl, 4-Hydroxyphenyl, 4-Brombenzyl, 4-tert-Butylphenyl, 2-Phenylethyl oder 3-Phenylpropyl sein; bevorzugt sind sie jedoch in dieser Bedeutung Phenyl oder Benzyl.

Wenn $R^1$ und $R^2$ zusammen oder $R^1$ und $R^3$ zusammen Alkylen sind, so können sie zusammen mit den C-Atomen, an die sie gebunden sind, einenCycloalkanring bilden, vorzugsweise einen Cyclopentan- oder Cyclohexanring, der substituiert sein kann durch $C_1$-$C_4$-Alkyl oder durch Gruppen -COOH oder -COOZ.

Wenn $R^1$ und $R^2$ zusammen eine direkte Bindung bilden, so stellen die Verbindungen der Formel II Ester oder Anhydride von ungesättigten Carbonsäuren dar.

Verbindungen der Formel II, die eine Gruppe -COOH enthalten können Basenadditionssalze bilden. Diese können Metallsalze, Ammoniumsalze oder Salze von organischen Aminen sein. Bevorzugt sind nichttoxischeSalze von Alkalimetallen, von Erdalkalimetallen, von Metallen der Gruppen II B, III A oder VIII des Periodensystems der Elemente, Ammoniumsalze und Salze von organischen Aminen. Beispiele hierfür sind Natrium-, Kalium-, Calcium-, Magnesium-, Zink-, Aluminium-, Ammonium-, Trialkylammonium- oder Tris(hydroxyethyl)ammonium-Salze.

Bevorzugte Verbindungen der Formel II sind solche, worin $R^1$, $R^2$, $R^3$ und $R^4$ Wasserstoff, $C_1$-$C_4$-Alkyl, -COOH, -COOZ oder durch -COOH oder -COOZ substituiertes $C_1$-$C_4$-Alkyl darstellen, insbesondere solche Verbindungen der Formel II, worin $R^4$ eine Gruppe -COOH oder -COOZ oder durch -COOH oder -COOZ

substituiertes Alkyl darstellt.

Besonders bevorzugt sind Verbindungen der Formel II, worin mindestens einer der Substituenten $R^1$, $R^2$, $R^3$ und $R^4$ -COOH oder eine durch -COOH substituierte Alkylgruppe ist und mindestens einer dieser Substituenten eine Gruppe -COOZ oder eine durch -COOZ substituierte Alkylgruppe ist. Unter diesen Halbester-Verbindungen sind solche bevorzugt, bei denen sich die Gruppen -COOH und -COOZ an zwei benachbarten C-Atomen befinden.

Spezifische Beispiele solcher Verbindungen sind:
Methyl-benzthiazol-2-ylthioacetat,
Phenyl-benzthiazol-2-ylthioacetat,
Ethyl-(5-trifluoromethylbenzthiazol-2-ylthio)acetat,
n-Propyl-(5-carboxybenzthiazol-2-ylthio)acetat,
n-Octyl-(5-ethoxycarbonylbenzthiazol-2-ylthio)acetat,
n-Dodecyl-(6-methylsulfonylbenzthiazol-2-ylthio)acetat,
Ethyl-3-(benzthiazol-2-ylthio)propionat,
Allyl-3-(benzthiazol-2-ylthio)propionat,
Cyclohexyl-3-(benzthiazol-2-ylthio)propionat,
n-Octadecyl-3-(benzthiazol-2-ylthio)propionat,
Ethyl-3-(benzthiazol-2-ylthio)-2-methyl-propionat,
iso-Butyl-4-(benzthiazol-2-ylthio)butyrate,
Ethyl-3-(benzthiazol-2-ylthio)butyrat,
2-Ethylhexyl-3-(benzthiazol-2-ylthio)butyrat,
2-Methoxyethyl-3-(benzthiazol-2-ylthio)-3-methylbutyrat,
Diisopropyl-benzthiazol-2-ylthiomalonat,
Dimethyl-benzthiazol-2-ylthiosuccinat,
Diethyl-benzthiazol-2-ylthiosuccinat,
Diisopropyl-benzthiazol-2-ylthiosuccinat,
Di-n-butyl-benzthiazol-2-ylthiosuccinat,
Di-i-octyl-benzthiazol-2-ylthiosuccinat,
Di-n-decyl-benzthiazol-2-ylthiosuccinat,
Di-n-octadecyl-benzthiazol-2-ylthiosuccinat,
Di-2-ethylhexyl-benzthiazol-2-ylthiosuccinat,
Dicyclohexyl-benzthiazol-2-ylthiosuccinat,
Diphenyl-benzthiazol-2-ylthiosuccinat,
Dibenzyl-benzthiazol-2-ylthiosuccinat,
Di-(4-methylphenyl)-benzthiazol-2-ylthiosuccinat,
Benzthiazol-2-ylthiobernsteinsäure-methyl-butyl-ester,
Benzthiazol-2-ylthiobernsteinsäurue-methyl-phenyl-ester,
Benzthiazol-2-ylthiobernsteinsäure-ethyl-benzyl-ester,
Benzthiazol-2-ylthiobernsteinsäure-monomethylester,
Benzthiazol-2-ylthiobernsteinsäure-mono(2-ethylhexyl)ester,
Di-n-propyl-5-methylbenzthiazol-2-ylthiosuccinat,
Di-n-hexyl-6-ethylbenzthiazol-2-ylthiosuccinat,
Di-but-3-enyl-4-isopropylbenzthiazol-2-ylthiosuccinat,
Methyl-allyl-(7-t-butylbenzthiazol-2-ylthio)succinat,
Dibenzyl-6-methoxybenzthiazol-2-ylthisuccinat,
5-Methoxybenzthiazol-2-ylthiobernsteinsäure-ethyl-methyl-ester,
Didodecyl-3-(benzthiazol-2-ylthio)-propan-1,2-dicarboxylat,
Diethyl-3(benzthiazol-2-ylthio)-propan-1,2-dicarboxylat,
Monoethyl-3(benzthiazol-2-ylthio)-propan-1,2-dicarboxylat,
Monobutyl-3-(benzthiazol-2-ylthio)-propan-1,2-dicarboxylat,
Monooctadecyl-3-(benzthiazol-2-ylthio)-propan-1,2-dicarboxylat,
Di-n-butyl-3-(6-carbmethoxybenzthiazol-2-ylthio)-propan-1,2-dicarboxylat,
Diethyl-1-(benzthiazol-2-ylthio)-propan-1,3-dicarboxylat,
Diisopropyl-2-(benzthiazol-2-ylthio)-propan-1,3-dicarboxylat,
Dimethyl-3-(benzthiazol-2-ylthio)-3-phenylpropan-1,2-dicarboxylat,
Diethyl-3-(benzthiazol-2-ylthio)-3-(4-carboxyphenyl)-propan-1,2-dicarboxylat,
Di-n-butyl-3-(benzthiazol-2-ylthio)-3-(2,4-dicarboxyphenyl)-propan-1,2-dicarboxylat,
Di-n-hexyl-3-(benzthiazol-2-ylthio)-3,3-diphenylpropan-1,2-dicarboxylat,

Dimethyl-1-(benzthiazol-2-ylthio)-butan-1,2-dicarboxylat,
Dibenzyl-1-(benzthiazol-2-ylthio)-2-methylpropan-1,2-dicarboxylat,
Di-allyl-2-(benzthiazol-2-ylthio)-butan-2,3-dicarboxylat,
Diphenyl-1-(benzthiazol-2-ylthio)-butan-2,4-dicarboxylat,
Trimethyl-4-(benzthiazol-2-ylthio)butan-1,2,3-tricarboxylat,
Dimethyl-hydrogen-4-(benzthiazol-2-ylthio)-butan-1,2,3-tricarboxylat,
Diethyl-1-(benzthiazol-2-yllthio)-pentan-1,5-dicarboxylat,
Di-n-hexyl-3-(benzthiazol-2-ylthio)-hexan-1,2-dicarboxylat,
Tetraethyl-8-(benzthiazol-2-ylthio)-octan-1,3,5,7-tetracarboxylat,
Dimethyl-1-(benzthiazol-2-ylthio)-cyclohexan-1,2-dicarboxylat,
Diphenyl-4-(benzthiazol-2-ylthio)-cyclohexan-1,2-dicarboxylat,
Tri-n-octyl-1-(benzthiazol-2-ylthio)-propan-1,2,3-tricarboxylat,
Di-n-pentyl-1-(benzthiazol-2-ylthio)-3-chlorpropan-1,2-dicarboxylat,
Di-n-nonyl-1-(benzthiazol-2-ylthio)-3-methoxypropan-1,2-dicarboxylat,
Di-n-decyl-1-(benzthiazol-2-ylthio)-3-hydroxypropan-1,2-dicarboxylat,
Dimethyl-1-(benzthiazol-2-ylthio)-2-phenylsuccinat,
Diethyl-1-(benzthiazol-2-ylthio)-2-benzylsuccinat,
Diethyl-2,3-bis-(benzthiazol-2-ylthio)-butan-1,4-dicarboxylat,
Natrium-monomethyl-benzthiazol-2-ylthiosuccinat,
Kalium-monoethyl-benzthiazol-2-ylthiosuccinat,
Calcium-monoethyl-benzthiazol-2-ylthiosuccinat,
Zink-monoisopropyl-benzthiazol-2-ylthiosuccinat,
Cobalt-monobutyl-benzthiazol-2-ylthiosuccinat,
Aluminium-mono-2-ethoxyethyl-benzthiazol-2-ylthiosuccinat,
Ammonium-mono-n-octyl-benzthiazol-2-ylthiosuccinat,
Methylammonium-monoethyl-benzthiazol-2-ylthiosuccinat,
Triethanolammonium-monomethyl-benzthiazol-2-ylthiosuccinat,
Octylammonium-monophenyl-benzthaizol-2-ylthiosuccinat,
Cyclohexylammonium-monobenzyl-benzthiazol-2-ylthiosuccinat,
Diethylammonium-mono-n-butyl-benzthiazol-2-ylthiosuccinat,
Tributylammonium-monomethyl-benzthiazol-2-ylthiosuccinat,
Natrium-monoethyl-3-(benzthiazol-2-ylthio)-propan-1,2-dicarboxylat,
Kalium-mono-n-propyl-3-(benzthiazol-2-ylthio)-propan-1,2-dicarboxylat,
Calcium-monomethyl-3-(benzthiazol-2-ylthio)-propan-1,2-dicarboxylat,
Zink-monobenzyl-3-(benzthiazol-2-ylthio)-propan-1,2-dicarboxylat,
Aluminium-monophenyl-3-(benzthiazol-2-ylthio)-propan-1,2-dicarboxylat,
Ammonium-monoallyl-3-(benzthiazol-2-ylthio)-propan-1,2-dicarboxylat,
Diisopropyl-1-(benzthiazol-2-ylthio)-propen-1,2-dicarboxylat,
Di-n-butyl-2-(benzthiazol-2-ylthio)-but-1-en-2,3-dicarboxylat,
2,2'-Bis(benzthiazol-2-ylthio)essigsäureanhydrid,
3,3'-Bis(benzthiazol-2-ylthio)propionsäureanhydrid,
Benzthiazol-2-ylthiobernsteinsäureanhydrid,
5-Methylbenzthiazol-2-ylthiobernsteinsäureanhydrid,
3-(Benzothiazol-2-ylthio)-propan-1,2-dicarbonsäureanhydrid,

Einige der erfindungsgemässen verwendbaren Korrosionsinhibitoren sind bereits in anderen Zusammenhängen bekannt geworden. So werden Verbindungen der Formel

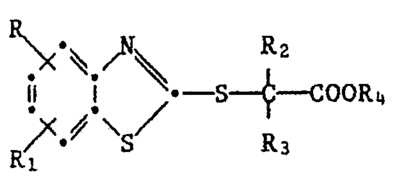

worin R und R' H, Alkyl, Alkoxy, OH, Alkanoyloxy, $NO_2$, $NH_2$, Alkanoylamino oder Halogen sind und $R_2$, $R_3$ und $R_4$ H oder Alkyl sind in der Jap. OS 79/3064, ref. Chem. Abstracts 90 (1979), 186930 beschrieben.

6-Alkoxybenzimidazol-2-ylthioglykolsäureester wurden als potentielle Baktericide im Indian J. Chem. 3 -

(1965), 377-401 , ref. Chem.Abstracts 64 (1966), 3519e, beschrieben.

Verbindungen der Formel

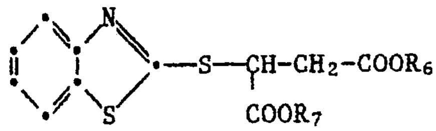

worin $R_6$ und $R_7$ gegebenenfalls durch 0 unterbrochene Kohlenwasserstoffreste mit 3-12 C-Atomen sind, sind im US-Patent 2,725.364 beschrieben.

Verbindungen der Formel

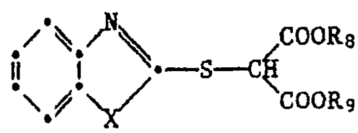

worin X Schwefel, Sauerstoff oder NH ist und $R_8$ und $R_9$ Alkylreste sind, werden in der Jap. OS 77/33532, ref. Chem. Abstracts. 88 (1978), 201015, beschrieben.

Verbindungen der Formel

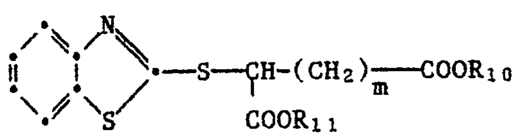

worin m 3 oder 4 ist und $R_{10}$ und $R_{11}$ Methyl oder Ethyl sind, sind im Bull. Chem. Soc. Japan 52 (1979), 267, beschrieben.

Andererseits sind viele der erfindungsgemäss verwendeten Korrosionsinhibitoren neue Verbindungen, die ebenfalls Gegenstand der Erfindung sind.

Dazu gehören die Halbester der Formel II, worin R, $R^1$, $R^2$, $R^3$, $R^4$, n und X die eingangs gegebene Bedeutung haben und die im Rest

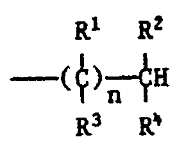

eine, zwei oder drei Gruppen -COOZ und mindestens eine Gruppe -COOH enthalten.

Eine zweite Gruppe von neuen Verbindungen sind die cyclischen Anhydride der Formel II, worin R, $R^1$, $R^2$, $R^3$, $R^4$, n und X die eingangs gegebene Bedeutung haben und die im Rest

$$-[C(R^1)(R^3)]_n-CH(R^2)(R^4)$$

eine oder zwei Gruppen der Formel III oder IIIa enthalten.

Die Verbindungen der Formel II können nach verschiedenen Methoden hergestellt werden. Eine sehr einfache Methode ist die Addition von $\alpha,\beta$-ungesättigten Carbonsäureestern de Formel V an 2-Mercaptobenzthiazole der Formel IV:

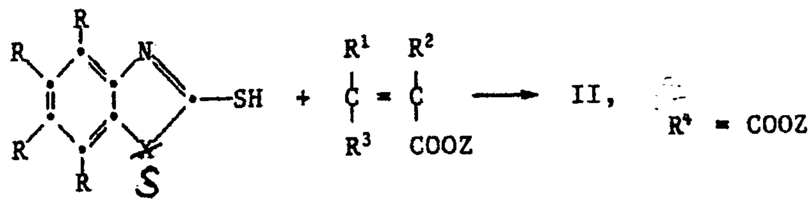

II, $R^4$ = COOZ

IV V

Diese Reaktion wird in stark saurem Medium ausgeführt und ist Gegenstand einer separaten Patentanmeldung.

Eine andere Methode ist die Umsetzung eines Benzazolderivates der Formel VI mit einem Mercaptocarbonsäurederivat der Formel VII

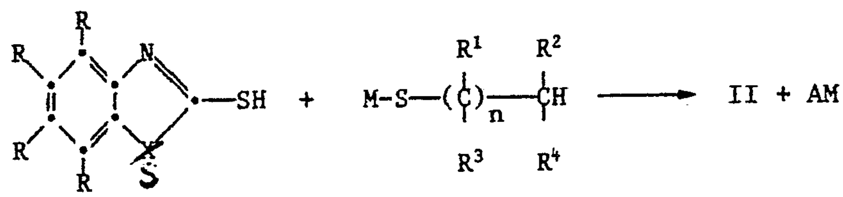

II + AM

VI VII

worin A eine Abgangsgruppe wie z.B. Cl, Br, I oder Tosyloxy ist, und M Wasserstoff oder ein Alkalimetall-, Erdalkalimetall- oder Ammonium-Kation ist.

Eine Variante dieser Methode ist die Umsetzung von Verbindungen der Formel VIII mit solchen der Formel IX

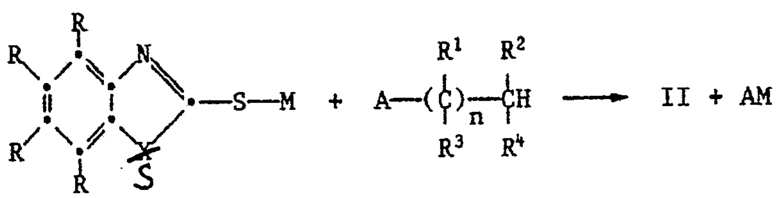

II + AM

VIII IX

Nach diesen Methoden können in einem ersten Schritt anstelle der Ester der Formel II auch die entsprechenden freien Carbonsäuren hergestellt werden, die in einem zweiten Schritt mit einer Verbindung Z-OH verestert werden. Es können auch zuerst Niederalkylester (Z = $C_1$-$C_4$-Alkyl) hergestellt werden, die in einem zweiten Schritt mit einer anderen Hydroxylverbindung Z-OH umgeestert werden.

Partielle Ester können durch partielle Hydrolyse der entsprechenden Vollester (von Polycarbonsäuren) hergestellt werden. Vorzugsweise stellt man sie jedoch durch Umsetzung der cyclischen Anhydride (Verbindungen der Formel II, die eine Gruppe III oder IIIa enthalten) mit einem Aequivalent Z-OH her.

Die cyclischen Anhydride der Formel II werden nach einer der vorhin genannten Methoden erhalten, wenn man als Komponente V, VII oder IX ein cyclisches Anhydrid verwendet. Sie können jedoch auch durch Dehydratisierung der entsprechenden Dicarbonsäuren erhalten werden. Geeignete Dehydratisierungsmittel sind z.B. Essigsäureanhydrid, Phosphorylchlorid oder Carbodiimide wie z.B. Dicyclohexylcarbodiimid. Durch Dehydratisierung können auch die erfindungsgemäss verwendbaren Anhydride von Monocarbonsäuren hergestellt werden.

Die bevorzugte Methode zur Herstellung cyclischer Anhydride der Formel II ist jedoch die Addition ungesättigter cyclischer Anhydride an Verbindungen der Formel IV. Hierfür geeignete Anhydride sind vor allem solche der Formel X und XI, deren Addition an IV zu den Anhydriden der Formel XII und XIII führt

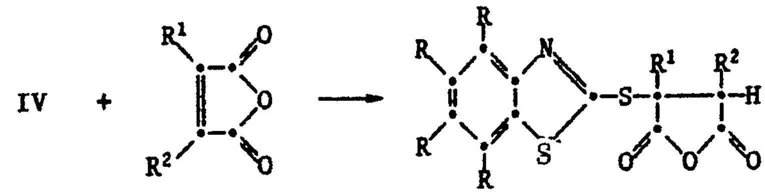

X XII

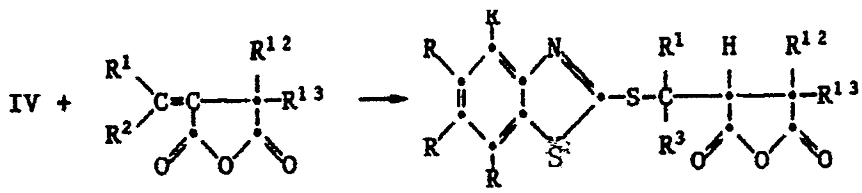

XI XIII

Darin bedeuten $R^{12}$ und $R^{13}$ Wasserstoff oder $C_1$-$C_{10}$-Alkyl, das durch ein oder zwei Gruppen -COOH oder -COOZ substituiert sein kann, wobei die Summe der C-Anzahl von $R^{12}$ und $R^{13}$ nicht grösser als 10 ist. Vorzugsweise sind die Substituenten $R^1$, $R^2$, $R^3$, $R^{12}$ und $R^{13}$ Wasserstoff. Verbindungen der Formel XII und XIII sind neue Verbindungen und als solche ebenfalls Gegenstand der Erfindung.

All diese Verbindungen mit der Gruppe der Formel I, insbesondere die Verbindungen der Formel II sind verwendbar als Korrosionsinhibitoren a) in Anstrichstoffen oder b) in wässrigen Systemen ausserhalb der Anstrichstoffe.

Die Anstrichstoffe enthalten als Hauptbestandteil ein Bindemittel das in organischer Lösung, in wässriger Dispersion oder Lösung oder in fester Form eingesetzt werden kann. Bindemittel für nichtwässrige Applikationen sind z.B. Epoxidharze (in Kombination mit einem Epoxid-Härter), Polyurethane, Aminoplaste, Acrylharze, Polyester oder Alkydharze und deren Mischungen. Weitere Beispiele für solche Bindemittel sind Phenolharze, Vinylharze wie Polyvinylchlorid , Polyvinylbutyral oder Polyvinylacetat und deren Copolymeren, Chlorkautschuk und andere chlorierte Harze, Styrol-Copolymerisate wie Styrol-Butadien, Celluloseester, Leinölharze und sonstige trocknenden Oele.

Für wässrige Anstrichstoffe eignen sich wasserlösliche oder wasserdispergierbare Bindemittel. Dies können beispielsweise Alkydharze, Polyester, Acrylharze, Polyurethane, Epoxidharze, Phenoplaste, Aminoplaste und deren Mischungen sein, weiterhin Homo-und Copolymere von Vinylethern, Vinylestern, Styrol, Vinylidenchlorid und Vinylchlorid. Die Härtung dieser wässrigen Anstrichmittel kann durch Vernetzung der Bindemittelharze mit z.B. Aminoplasten, Phenoplasten, blockierten Isocyanaten, Epoxidharzen, aktivierten Carbonsäureestern oder Mannichbasen von Phenolen bewirkt werden. Die für wässrige Anstrichstoffe verwendeten Bindemittel sind oft durch Einbau basischer oder saurer Gruppen modifizierte Harze. Durch Neutralisation dieser Gruppen werden diese Harze wasserlöslich oder wasser-dispergierbar.

Die Anstrichstoffe können ausser dem Bindemittel und dem erfindungsgemässen Korrosionsinhibitor weitere Komponenten enthalten, wie z.B. Pigmente, Farbstoffe, Füllstoffe oder andere Additive wie sie für Anstrichstoffe üblich sind. Das Pigment kann ein organisches, anorganisches oder metallisches Pigment sein, z.B. Titandioxid, Eisenoxid, Aluminiumbronze, Phthalocyaninblau etc. Man kann zusätzlich auch antikorrosive Pigmente verwenden, wie z.B. Phosphat- oder Borat-enthaltende Pigmente, Metallpigmente oder Metalloxidpigmente, wie sie in Farbe und Lack 88, 183 (1982) genannt sind oder wie sie im EP-A1-54267 aufgeführt sind. Beispiele für verwendbare Füllstoffe sind Talkum, Kalk, Aluminiumoxid, Baryt, Glimmer oder Kieselerden. Beispiele für sonstige Zusatzstsoffe sind Verlaufhilfsmittel, Dispersionsmittel, Thixotropiemittel, Haftverbesserer, Antioxydantien, Lichtschutzmittel oder Härtungskatalysatoren.

Von besonderer Bedeutung ist der Zusatz basischer Füllstoffe oder Pigmente. Diese bewirken in bestimmten Bindemittelsystemen wie z.B. in Acryl- und Alkydharzen einen synergistischen Effekt auf die Korrosionsinhibierung. Beispiele für solche basische Füllstoffe oder Pigmente sind Calcium- oder Magnesiumcarbonat, Zinkoxid, Zinkcarbonat, Zinkphosphat, Magnesiumoxid, Aluminiumoxid, Aluminiumphosphat oder Gemische davon. Beispiele für Pigmente sind z.B. solche auf Basis von Aminoanthrachinon.

Die erfindungsgemässen Korrosionsinhibitoren können auch zuerst auf solche basische Füllstoffe oder Pigmente aufgebracht werden, beispielsweise durch Chemisorption aus wässriger Lösung, und die so

erhaltenen Zubereitungen dem Anstrichstoff zugesetzt werden.

In einer weiteren bevorzugten Ausführungsform der Erfindung werden die Korrosionsinhibitoren zusammen mit basisichen Ionenaustauschern verwendet oder man behandelt einen solchen Ionenaustauscher zuerst mit einer Lösung des Inhibitors und setzt diese Zubereitung dem Ueberzugsmittel zu. Beispiele für basische Ionenaustauscher sind alle typischen Anionenaustauscher, wie sie z.B. unter den Namen Dowex® 1 oder 11 oder Amberlite® IRA im Handel sind.

Schliesslich kann der Korrosionsinhibitor auch auf einem neutralen Trägerstoff aufgebracht werden. Als Trägerstoffe eignen sich insbesondere pulverförmige Füllstoffe oder Pigmente. Diese Technik ist in der DE-OS 31 22 907 näher beschrieben.

Der Anstrichstoff kann ausser dem erfindungsgemässen Korrosionsinhibitor noch andere organische, metallorganische oder anorganische Korrosionsinhibitoren enthalten wie z.B. Salze der Nitroisophthalsäure,Tannin, Phosphorester, technische Amine, substituierte Benztriazole oder substituierte Phenole, wie sie z.B. in der DE-OS 31 46 265 beschrieben sind.

Die Korrosionsinhibitoren können dem Anstrichstoff während dessen Herstellung zugesetzt werden, z.B. während der Pigmentverteilung durch Mahlen oder man löst die Inhibitoren in einem Lösungsmittel vor und rührt die Lösung in den Anstrichstoff ein. Man verwendet den Inhibitor in einer Menge von 0,1 - 20 Gew.-%, vorzugsweise 0,5 - 5 Gew.-%, bezogen auf den Feststoffgehalt des Anstrichstoffes.

Die Erfindung betrifft daher auch antikorrosive Anstrichstoffe, die als Korrosionsinhibitor 0,1 -20 Gew.-% (bezogen auf den Feststoffgehalt) eines Esters oder Anhydrides einer aliphatischen oder cycloaliphatischen Mono-, Di-, Tri- oder Tetracarbonsäure enthalten, welche im aliphatischen oder cycloaliphatischen Rest durch eine oder mehrere, vorzugsweise durch eine Gruppe der Formel I substituiert ist,

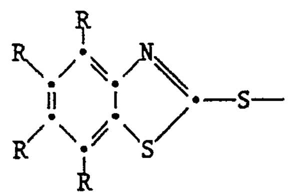

(I)

worin einer der Substituenten R Wasserstoff, $C_1$-$C_4$-Alkyl oder $C_1$-$C_4$-Alkoxy ist und die anderen drei R Wasserstoff sind oder eines nicht-toxischen Salzes einer solchen Verbindung, die eine Carboxylgruppe enthält.

Die erfindungsgemässen Anstrichstoffe werden vorzugsweise als Grundierung (Primer) auf metallischen Substraten, insbesondere auf Eisen, Stahl, Kupfer und Aluminium verwendet. Sie können dabei als sogenannte Konversions-Anstriche fungieren, indem an der Grenzfläche von Metall und Ueberzug chemische Reaktionen stattfinden. Der Auftrag der Ueberzüge kann nach den üblichen Methoden erfolgen wie Spritzen, Streichen, Aufrollen, Tauchen oder durch Elektroabscheidung, insbesondere kathodische Abscheidung. Je nachdem, ob der Filmbildner ein physikalisch trocknendes oder ein hitze- oder strahlenhärtbares Harz ist, erfolgt die Aushärtung der Ueberzüge bei Raumtemperatur, durch Einbrennen oder durch Bestrahlung.

Die erfindungsgemässen Korrosionsinhibitoren können auch in wässrigen Systemen ausserhalb der Anstrichstoffe verwendet werden. Dies können rein-wässrige oder teil-wässrige Systeme sein. Beispiele für rein-wässrige Systeme sind Kühlwasser-Systeme, Klimaanlagen, Dampfgeneratoren, Seewasserverdampfer oder Heizwasser-Kreisläufe. Wenn solche Systeme im Kontakt mit Metallen stehen, benötigen sie den Zusatz eines Korrosionsinhibitors. Die erfindungsgemässen Korrosionsinhibitoren werden in einer Menge von 0,1 bis 50000 ppm (= 0,00001 bis 5 %), vorzugsweise 1 bis 500 ppm (= 0,0001 bis 0,05 %) zugesetzt. Sie können allein oder in Kombination mit bekannten Korrosionsinhibitoren zugesetzt werden. Bekannte Inhibitoren für wässrige Systeme sind z.B. wasserlösliche Zinksalze, Phosphate, Polyphosphate, Phosphonsäuren und deren Salze wie z.B. Acetodiphosphonsäure, Nitrilotrimethylenphosphonsäure oder Methylamino-dimethylenphosphonocarbonsäuren und ihre Salze, beispielsweise solche, wie sie in der DE-OS 2,632,774 beschrieben sind, Phosphonohydroxyessigsäure, 2-Phosphonobutan-1,2,4-tricarbonsäure oder die in GB-A-1,572,406 beschriebenen Säuren. Weitere mitverwendbare bekannte Korrosionsinhibitoren für wässrige Systeme sind Nitrate (z.B. $NaNO_3$, Nitrite (z.B. $NaNO_2$), Molybdate, Wolframate, Silicate, Benztriazol, Tolutriazol und deren Derivate, N-Acylsarcosine, N-Acyliminodiessigsäuren, Ethanolamine, Fettamine, Polycarbonsäuren wie z.B. Polymerisate und Copolymerisate der Acrylsäure oder der Maleinsäure.

Die wässrigen Systeme können weitere Zusätze enthalten wie Komplexbildner (z.B. Nitrilotriessigsäu-

re), Reduktionsmittel (z.B. Alkalisulfite oder Hydrazin), Antischaum-mittel (z.B. Silicone oder Fettalkohol-Ethylenoxid-Addukte) oder Biocide (z.B. Quaternäre Ammoniumverbindusngen, Chlor-abgehende Verbindungen oder zinnorganische Verbindungen).

Teil-wässrige Systeme, in denen die erfindungsgemässen Korrosionsinhibitoren eingesetzt werden können, sind hydraulische Flüssigkeiten, Gefrierschutzmittel oder Metallbearbeitungs-Flüssigkeiten, die z.B. beim Bohren, Fräsen, Schneiden, Sägen, Schleifen oder Verformen von Metallen verwendet werden. Diese enthalten meist eine emulgierte ölige Phase, können aber auch wasserlösliche Systeme sein, z.B. auf Basis von Polyethylenglykolen. Sie werden oft als Konzentrate in den Handel gebracht, die vor Gebrauch mit Wasser verdünnt werden. Die Zusatzmengen an Korrosionsinhibitor sind dieselben wie bei den rein-wässrigen Systemen. Bei den Konzentraten sind Zusammensetzusngen von 0,05 bis 5 % bevorzugt, dies entspricht in den verdünnten Zubereitungen etwa 5 bis 1000 ppm.

Auch in diesen Systemen können die erfindungsgemässen Korrosionsinhibitoren zusammen mit anderen bekannten Inhibitoren verwendet werden. Solche sind z.B. organische Säuren und deren Salze (z.B. Benzoesäure, Dinatrium-sebacat, Triethanolamin-laurat oder Natrium-N-lauroylsarcosinat), organische Stickstoff-Verbindungen (wie z.B. Fettsäurealkanolamide, Imidazoline, Oxazoline, Benztriazole, Fettamine, Alkanolamine), anorganische Nitrite (z.B. $NaNO_2$), organische Phosphorverbindungen (z.B. Phosphonsäuren oder Aminophosphate), anorganische Phosphate (z.B. $NaH_2PO_4$ oder Zinkphosphat) oder organische Schwefelverbindungen und deren Salze (z.B. Salze von Petroleumsulfonaten oder Natrium-Mercaptobenzthiazol). Bevorzugt ist die Mitverwendung von Triethanolamin.

Die folgenden Beispiele illustrieren die vorliegende Erfindung näher. Darin bedeuten Teile und % Gewichtsteile und Gewichts.-% sofern nicht anders angegeben. Die Temperaturen sind in °C angegeben.

Beispiel 1: Herstellung der Vollester durch Veresterung der Carbonsäuren

A) Eine Lösung von 39,4 g Brombernsteinsäure in 200 ml 10 %-iger $Na_2CO_3$-Lösung wird innerhalb 30 min zu einer Lösung von 41,5 g 2-Mercaptobenzthiazol in 200 ml 10%iger $Na_2CO_3$-Lösung bei 80° unter Rühren zugegeben und die Mischung weitere 2 h auf 80° erwärmt. Die entstehende Suspension wird nach dem Abkühlen mit Chloroform extrahiert um nicht-umgesetztes Mercaptobenzthiazol zu entfernen. Die wässrige Phase wird mit konzentrierter Salzsäure angesäuert und mit Diethylether extrahiert. Die Etherlösung wird eingedampft und der Rückstand aus wässrigem Methanol umkristallisiert. Man erhält so die Benzthiazol-2-yl-bernsteinsäure, die bei 175-178° unter Zersetzung schmilzt.

NMR-Spektrum ($\delta$ DMSO-$d_6$): 3,15 (d,2H), 4,95 (t,1H), 7,20-8,20 (c,4H).

B) Eine Lösung von 10 g der Benzthiazolylthiobernsteinsäsure in 150 ml absolutem Ethanol wird bei Raumtemperatur mit HCl-Gas gesättigt und 10 h stehen gelassen. Dann wird das Ethanol im Vakuum abdestilliert und das zurückbleibende Oel durch Kurzwegdestillation im Hochvakuum gereinigt. Man erhält den Benzthiazol-2-ylthiobernsteinsäure-diethylester (Verbindung Nr. 1) als ölige Flüssigkeit, dessen NMR-Spektrum mit der Struktur des Produktes übereinstimmt und folgende Banden zeigt: 1,15 (t,3H); 3,25 (d,2H); 4,20 (m,4H); 5,10 (t,1H); 7,45 (m,2H); 8,05 (m,2H).

In analoger Weise erhält man den Benzthiazol-2-ylthiobernsteinsäure-dibutylester (Verbindung Nr. 2), der ebenfalls eine ölige Flüssigkeit ist. NMR-Spektrum ($\delta$ $CCl_4$): 0,95 (t,6H); 1,55 (m,8H); 3,15 (d,2H); 4,15 (m,4H); 5,00 (t,1H); 7,30 (m,2H); 7,80 (m,2H).

Benzthiazol-2-ylthiobernsteinsäure-di(2-ethylhexyl)ester (Verbindung Nr: 3) wird in analoger Weise hergestellt, wobei jedoch die Veresterung bei 85° durchgeführt wird.

NMR-Spektrum ($\delta$ $CDCl_3$): 1,0 (t,12H); 1,5 (m,18H); 3,3 (d,2H); 4,2 (m,4H); 5,2 (t,1H); 7,2 (m,2H); 7,8 (m,2H).

In analoger Weise werden hergestellt:

Benzthiazol-2-ylthiopropionsäure-cyclohexylester (Verbindung Nr. 4) aus Benzthiazol-2-ylthiopropionsäure und Cyclohexanol durch HCl-katalysierte Veresterung bei 95°.

3-(Benzthiazol-2-ylthio)-propan-1,2-dicarbonsäure-diethylester (Verbindung Nr. 5) aus der Säure und Ethanol bei Rückflusstemperatur. NMR-Spektrum ($\delta$ $CDCl_3$): 1,4 (t,6H); 3,0 (d,2H); 3,5 (m,1H); 3,8 (m,2H); 4,2 (m,4H); 7,2 (m,2H); 7,7 (m,2H).

3-(Benzthiazol-2-ylthio)-propan-1,2-dicarbonsäure-di-n-dodecylester (Verbindung Nr. 6) aus der Säure und 1-Dodecanol bei 85°. NMR-Spektrum ($\delta$ $CDCl_3$): 1,0 (t,6H); 1,4 (m,40H); 3,0 (d,2H); 3,6 (m,1H); 3,9 (m,2H); 4,2 (m,4H); 7,3 (m,2H); 7,8 (m,2H).

Beispiel 2: Herstellung von Anhydriden

Eine Lösung von 6,18 g Dicyclohexylcarbodiimid in 30 ml 1,2-Dimethoxyethan wird tropfenweise unter

Rühren zu einer Lösung von 8,49 g Benzthiazol-2-ylthiobernsteinsäure in 70 ml Dimethoxyethan zugegeben. Die Reaktion ist exotherm und die Temperatur wird durch Kühlung auf 25-27° gehalten. Nach Beendigung der Zugabe rührt man weitere 1,5 h und filtriert dann den ausgefallenen Dicyclohexylharnstoff ab. Das Filtrat wird im Vakuum eingedampft und der Rückstand aus einer Mischung von Dichlormethan und Cyclohexan umkristallisiert. Man erhält so das Benzthiazol-2-ylthiobernsteinsäureanhydrid (Verbindung Nr. 7), das bei 128-9° unter Zersetzung schmilzt.

| | | | | |
|---|---|---|---|---|
| Analyse ($C_{11}H_7NO_3S_2$) | ber. | C 48,71 % | H 2,67 % | N 5,28 % |
| | gef. | C 49,76 % | H 2,53 % | N 5,40 %. |

Eine Suspension von 20 g 3-(Benzthiazol-2-ylthio)-propan-1,2,-dicarbonsäure in 200 g Essigsäureanhydrid wird 1,5 h auf 55° erwärmt. Die resultierende gelbe Lösung wird nach dem Abkühlen auf 1 l Eiswasser gegossen, wobei das Dicarbonsäureanhydrid fest ausfällt. Es wird abfiltriert und im Vakuum getrocknet (Verbindung Nr. 8). Smp. 87-88°. NMR-Spektrum (δ $CDCl_3$): 3,05 (d,2H); 3,65 (m,3H): 7,15 (m,2H);7,6 (m,2H).

Beispiel 3: Herstellung der Halbester

Ein Gemisch von 6,5 g Benzthiazol-2-ylthiobernsteinsäureanhydrid (Verbindung Nr. 7) und 75 ml absolutem Ethanol wird 10 Minuten zum Rückfluss erhitzt, wobei eine klare Lösung resultiert. Diese wird 13 h bei Raumtemperatur gerührt und dann im Vakuum eingedampft. Der Rückstand wird in 80 ml $CHCl_3$ aufgenommen und mit konzentrierter $NaHCO_3$-Lösung extrahiert. Die wässrige Phase wird mit Tierkohle geschüttelt, filtriert und mit konzentrierter HCl angesäuert. Das ausgefallene Oel wird mit Diethylether extrahiert, die Etherlösung mit Wasser gewaschen, über $MgSO_4$ getrocknet und eingedampft. Das Produkt wird weiter gereinigt durch Chromatographie an Kieselgel (Elution mit Ethylacetat) und man erhält Benzthiazol-2-ylthiobernsteinsäure-monoethylester (Verbindung Nr. 9) als ölige Mischung beider möglicher Isomeren.

| | | | | |
|---|---|---|---|---|
| Analyse ($C_{13}H_{13}NO_4S_2$) | ber. | C 50,16 % | H 4,22 % | N 4,50 % |
| | gef. | C 50,04 % | H 4,26 % | N 4,34 % |

In analoger Weise erhält man aus dem oben angeführten Anhydrid und 2-Ethylhexanol ein öliges Isomerengemisch des Benzthiazol-2-ylthiobernsteinsäure-mono(2-ethylhexyl)esters (Verbindung Nr. 10)

| | | | | |
|---|---|---|---|---|
| Analyse ($C_{19}H_{25}NO_4S_2$) | ber. | C 57,70 % | H 6,38 % | N 3,54 % |
| | gef. | C 57,81 % | H 6,27 % | N 3,35% |

Analog erhält man den Monomethylester (Verbindung Nr. 11) NMR-Spektrum (δ $CDCl_3$): 3,10 (d) und 3,25 (d)(2H); 3,70 (s) und 3,75 (s)(3H); 4,85 (1) und 4,95 (t)(1H); 7,45 (m,2H), 7,85 (m,4H); 10,75 (s,1H).

Benzthiazol-2-ylthiobernsteinsäure-monobutylester (Verbindung Nr. 12) als öliges Isomerengemisch. NMR-Spektrum (δ $CDCl_3$): 0,85 (6,3H); 1,45 (m,4H); 3,15 (d,2H); 4,15 (m,2H); 4,85 (t,1H); 7,18 (m,2H); 7,60 (m,2H); 9,19 (s,1H).

Benzthiazol-2-ylthiobernsteinsäure-mono-n-octadecylester (Verbindung Nr. 13) als öliges Isomerengemisch.

| | | | | | |
|---|---|---|---|---|---|
| Analyse ($C_{29}H_{45}NO_4S_2$) | ber. | C 65,05 % | H 8,41 % | N 2,62 % | S 11,96 % |
| | gef. | C 65,45 % | H 8,80 % | N 2,53 % | S 11,83 % |

3-(Benzthiazol-2-ylthio)-propan-1,2-dicarbonsäure-monobutylester (Verbindung Nr. 14) aus Verbindung Nr. 8 und n-Butanol bei 55°/7h als Rohprodukt (braunes Oel).

NMR-Spektrum (δ-$CDCl_3$): 0,85 (m,3H); 2,8 (d, 2H); 3,35 (m,1H); 3,6 (m,2H); 4,04 (m,2H); 7,2 (m,2H); 7,62, (m,2H).

3-(Benzthiazol-2-ylthio)-propan-1,2-dicarbonsäure-mono-n-octadecyles ter (Verbindung Nr. 15) in analoger Weise als öliges Isomerengemisch.
NMR-Spektkrum (δ $CDCl_3$): 0,85 (b,3H); 1,25 (s,32H): 2,9 (d,2H); 3,42 (m, 1H); 3,71 (m, 2H); 4,1 (m,2H); 7,15 (m,2H), 7,8 (m,2H).

Beispiel 4: Benzthiazol-2-ylthioessigsäure-ethylester

55 g 2-Mercaptobenzthiazol werden in 450 ml Methanol bei 45° gelöst. Dazu wird eine Lösung von 7,8 g Natrium in 60 ml trockenem Methanol langsam unter Rühren zugetropft. Dann werden innerhalb 10 min 42 g Chloressigsäure-ethylester zugegeben und das Gemisch 5 h zum Rückfluss erhitzt. Nach dem Abkühlen gibt man 200 ml Wasser und 300 ml Toluol zu, trennt die Toluolphase ab, wäscht sie mit Wasser, trocknet sie über $Na_2SO_4$ und dampft sie im Vakuum ein. Es hinterbleibt der rohe Ethylester (Verbindung Nr. 16) als oranges Oel, das beim Stehen kristallisiert, Smp. 45-48°.

Beispiel 5: Herstellung von Estern durch Addition ungesättigter Ester

Zu einer Suspension von 16,8 g feingepulvertem 2-Mercaptobenzthiazol in 150 ml 70 %iger Schwefelsäure werden bei 0° bis 10° innerhalb einer Stunde 11,0 g Acrylsäure-ethylester unter Rühren zugetropft. Nach weiteren 1,5 h Rühren bei 0-10° wird das Reaktionsgemisch in Eiswasser gegossen und mit Ethylacetat extrahiert. Durch Eindampfen der organischen Phase erhält man den 3-(Benzthiazol-2-ylthio)-propionsäure-ethylester (Verbindung Nr. 17) als Flüssigkeit,
$n_D^{20}$ = 1,6120.

| | | | |
|---|---|---|---|
| Analyse ($C_{12}H_{13}NO_2S_2$) | ber. | N 5,2 % | S 24,0 % |
| | gef. | 5,3 % | S 24,0 % |

Analog wird aus Mercaptobenzthiazol und Crotonsäure-ethylester der 3(Benzthiazol-2-ylthio)-buttersäure-ethylester (Verbindung Nr. 18)
als ölige Flüssigkeit ($n_D^{20}$ = 1,5965) erhalten.

| | | | |
|---|---|---|---|
| Analyse ($C_{13}H_{15}NO_2S_2$) | ber. | N 5,0 % | S 22,8 % |
| | gef. | N 5,0 % | S 22,5 % |

Analog erhält man aus Mercaptobenzthiazol und Itaconsäure-dimethylester den 3-(Benzthiazol-2-ylthio)-propan-1,2-dicarbonsäure-dimethylester (Verbindung Nr. 19), der bei 46-47° schmilzt.

| | | | |
|---|---|---|---|
| Analyse ($C_{14}H_{15}NO_4S_2$) | ber. | N 4,3 % | S 19,7 % |
| | gef. | N 4,2 % | S 19,5 %. |

Beispiel 6: Korrosionsschutz in einem Alkydharz-Lack

Ein Alkydharzlack wird nach folgender Rezeptur bereitet:

| | |
|---|---|
| 40 | Teile Alphthalat® AM 380 (60 %ige Xylol-Lösung) Alkydharz der Fa. Reichhold Albert Chemie AG |
| 4 | Teile Eisenoxidrot 225 der Fa. Bayer AG |
| 17,4 | Teile Talkum (mikronisiert) |
| 13 | Teile mikronisiertes Calciumcarbonat (Millicarb®, Plüss-Staufer AG) |
| 0,3 | Teile Hautverhütungsmittel Luaktin® (BASF) |
| 0,6 | Teile 8 %ige Cobaltnaphthenat-Lösung |

24,7 Teile Xylol/Ethylglykol-Gemisch 6.40.

Die in der folgenden Tabelle angegebenen Korrosionsinhibitoren werden in einem Teil des Lösungsmittels vorgelöst und dem Lack zugegeben. Der Lack wird 7 Tage mit Glasperlen gemahlen bis zu einer Pigment- und Füllstoffkorngrösse von 15 μm.

Der Lack wird auf sandgestrahlte Stahlbleche von 7 x 13 cm aufgespritzt in einer Schichtdicke, die nach dem Trocknen ca. 50 μm beträgt. Nach 7 Tagen Trocknung bei Raumtemperatur werden die Proben 60 Minuten bei 60 °C ausgehörtet.

In die gehärtete Lackoberfläche werden zwei kreuzförmige Schnitte von 4 cm Länge bis zum Metall eingeschnitten mit Hilfe eines Bonder-Kreuzschnittgerätes. Zum Schutze der Kanten wird auf diese ein Kantenschutzmittel (Icosit® 225) aufgebracht.

Die Proben werden nunmehr einem Salzsprühtest gemäss ASTM B 117 mit einer Dauer von 600 Stunden unterworfen. Nach jeweils 200 Stunden Bewitterung wird der Zustand des Anstriches beurteilt, und zwar der Blasengrad (gemäss DIN 53 209) am Kreuzschnitt und auf der lackierten Fläche sowie der Rostgrad (gemäss DIN 53210) auf der ganzen Fläche.

Nach Ende des Tests wird der Anstrich durch Behandlung mit konzentrierter Natronlauge entfernt und die Korrosion des Metalls am Kreuzschnitt (gemäss DIN 53 167) sowie über die restliche Fläche beurteilt. Die Beurteilung erfolgt jeweils in einer 6-Stufen-Skala.

Die Sumse der Bewertung des Anstriche und der Bewertung der Metalloberfläche ergibt den Korrosionsschutzwert KS. Je höher dieser ist, desto wirkungsvoller ist der getestete Inhibitor.

Tabelle 1: Korrosionsinhibitoren der Formel

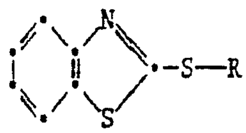

in Alkydharz-Lack

| Verbindung Nr. | R | Zusatzmenge | Beurteilung Anstrich | Metall | KS |
|---|---|---|---|---|---|
| 1 | $-CH-CH_2-COOC_2H_5$<br>$COOC_2H_5$ | 2 %<br>4 % | 3,3<br>2,3 | 2,7<br>1,9 | 6,0<br>4,2 |
| 2 | $-CH-CH_2-COOC_4H_9$<br>$COOC_4H_9$ | 2 %<br>4 % | 3,2<br>3,0 | 3,2<br>3,6 | 6,4<br>6,6 |
| 3 | $-CH-CH_2-COOC_8H_{17}$<br>$COOC_8H_{17}$ | 2 %<br>4 % | 3,6<br>2,4 | 1,7<br>0,8 | 5,3<br>3,2 |
| 19 | $-CH_2-CH-CH_2-COOCH_3$<br>$COOCH_3$ | 2 %<br>4 % | 3,5<br>3,7 | 2,9<br>4,3 | 6,4<br>8,0 |
| 5 | $-CH_2-CH-CH_2-COOC_2H_5$<br>$COOC_2H_5$ | 2 %<br>4 % | 3,6<br>3,9 | 3,8<br>3,6 | 7,4<br>7,5 |
| 6 | $-CH_2-CH-CH_2-COOC_{12}H_{25}$<br>$COOC_{12}H_{25}$ | 2 %<br>4 % | 3,1<br>3,3 | 1,8<br>3,2 | 4,9<br>6,5 |
| 4 | $-CH_2-CH_2-COO-$ 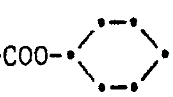 | 2 %<br>4 % | 4,6<br>3,6 | 3,2<br>1,7 | 7,8<br>5,3 |

| Verbindung Nr. | R | Zusatzmenge | Beurteilung Anstrich | Metall | KS |
|---|---|---|---|---|---|
| 16 | $-CH_2-COOC_2H_5$ | 2 %<br>4 % | 4,3<br>4,4 | 3,7<br>3,7 | 8,0<br>8,1 |
| 17 | $-CH_2-CH_2-COOC_2H_5$ | 2 %<br>4 % | 1,8<br>1,7 | 0,6<br>0,6 | 2,4<br>2,3 |
| 18 | $-CH-CH_2-COOC_2H_5$<br>$CH_3$ | 2 %<br>4 % | 3,6<br>3,2 | 3,0<br>2,7 | 6,6<br>5,9 |
| 7 | 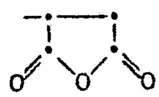 | 2 %<br>4 % | 2,8<br>0,6 | 0,6<br>0,6 | 3,4<br>1,2 |
| 8 | 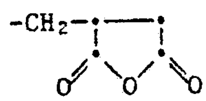 | 2 %<br>4 % | 4,4<br>4,0 | 4,3<br>2,8 | 8,7<br>6,8 |
| 9 | $-CH-CH_2-COOC_2H_5$<br>$COOH$<br>+<br>$-CH-CH_2-COOH$<br>$COOC_2H_5$ | 2 %<br>4 % | 4,2<br>2,4 | 4,0<br>2,5 | 8,2<br>4,9 |
| 11 | $-CH-CH_2-COOCH_3$<br>$COOH$<br>+<br>$-CH-CH_2-COOH$<br>$COOCH_3$ | 2 %<br>4 % | 4,4<br>2,2 | 1,8<br>0,6 | 6,2<br>2,8 |
| 12 | $-CH-CH_2-COOC_4H_9$<br>$COOH$<br>+<br>$-CH-CH_2-COOH$<br>$COOC_4H_9$ | 2 %<br>4 % | 4,4<br>4,5 | 4,0<br>3,5 | 8,4<br>8,0 |
| 13 | $-CH-CH_2-COOC_{18}H_{37}$<br>$COOH$<br>+<br>$-CH-CH_2-COOH$<br>$COOC_{13}H_{37}$ | 2 %<br>4 % | 4,5<br>3,7 | 4,6<br>1,7 | 9,1<br>5,4 |
| 14 | $-CH_2-CH-CH_2-COOC_4H_9$<br>$COOH$<br>+<br>$-CH_2-CH-CH_2-COOH$<br>$COOC_{18}H_{37}$ | 2 %<br>4 % | 3,5<br>4,4 | 3,3<br>5,0 | 6,8<br>9,4 |
| 15 | $-CH_2-CH-CH_2-COOC_{18}H_{37}$<br>$COOH$<br>+<br>$-CH_2-CH-CH_2-COOH$<br>$COOC_{18}H_{37}$ | 2 %<br>4 % | 4,3<br>4,0 | 3,5<br>3,0 | 7,8<br>7,0 |
| | ohne Inhibitor | - | 1,4 | 1,0 | 2,4 |

## Ansprüche

1. Verwendung von Estern oder Anhydriden aliphatischer oder cycloaliphatischer Mono-, Di-, Tri- oder Tetracarbonsäuren, die im (cyclo-)aliphatischen Rest durch eine oder mehrere der Gruppen der Formel

I substituiert sind,

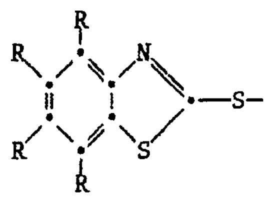

I

worin einer der Substituenten R Wasserstoff, $C_1$-$C_4$-Alkyl oder $C_1$-$C_4$-Alkoxy ist und die drei anderen R Wasserstoff sind, oder von nicht-toxischen Salzen solcher Verbindungen als Korrosionsinhibitoren.

2. Verwendung gemäss Anspruch 1 von Estern und Anhydriden, die die Gruppe I enthalten, worin alle R Wasserstoff sind.

3. Verwendung gemäss Anspruch 1 von Estern oder Anhydriden der Formel II,

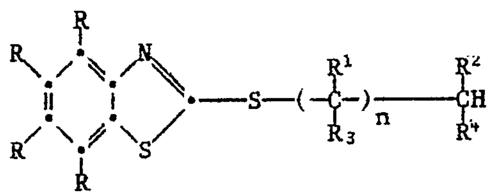

II

worin R die in Anspruch 1 gegebene Bedeutung hat, n Null oder 1 ist und $R^1$, $R^2$, $R^3$, $R^4$ unabhängig voneinander Wasserstoff, $C_1$-$C_{18}$-Alkyl, $C_1$-$C_4$-Hydroxyalkyl, $C_1$-$C_4$-Halogenalkyl, $C_2$-$C_{10}$-Alkoxyalkyl, unsubstituiertes oder im Phenylkern durch Halogen oder Hydroxy substituiertes Phenyl oder Phenylalkyl, eine Gruppe -COOH oder -COOZ oder durch 1, 2 oder 3 Gruppen -COOH oder -COOZ substituiertes $C_2$-$C_{12}$-Alkyl bedeuten, worin Z $C_1$-$C_{18}$-Alkyl bedeutet, das durch ein oder mehrere O- oder S-Atome oder $NR^{\circ}$ -Gruppen unterbrochen sein kann (worin $R^{\circ}$ $C_1$-$C_{18}$-Alkyl, $C_5$-$C_8$-Cycloalkyl, Phenyl, Naphthyl, $C_7$-$C_9$-Phenylalkyl oder $C_7$-$C_{18}$-Alkylphenyl bedeutet) oder durch -SH, $-COOR^{\circ}$, $-CONH_2$, -CN oder Halogen substituiert sein kann, oder Z $C_2$-$C_{10}$-Hydroxyalkyl bedeutet (das durch eine oder mehrere $NR^{\circ}$-Gruppen oder O-Atome unterbrochen sein kann), oder $C_2$-$C_{18}$-Alkenyl, $C_3$-$C_{12}$-Cycloalkyl (das durch $C_1$-$C_4$-Alkyl, -OH, -SH, $-COOR^{\circ}$, $-CONH_2$, -CN oder Halogen substituiert sein kann), $C_7C_9$-Phenylalkyl, $C_7$-$C_{18}$-Alkylphenyl oder $C_6$-$C_{10}$-Aryl (das durch $C_1$-$C_{12}$-Alkoxy, $c_1$-$c_{12}$-Alkylthio, -COOH, -OH, $-NO_2$ oder Halogen substituiert sein kann) bedeutet, sowie solche Verbindungen der Formel II, worin im Rest

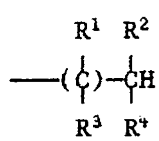

zwei Gruppen -COOH zu einer Anhydridgruppe der Formel III oder IIIa cyclisiert sind,

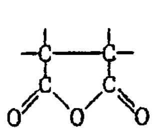

III

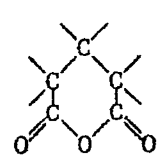

IIIa

sowie solche Verbindungen der Formel II, worin $R^1$ und $R^2$ oder $R^1$ und $R^3$ zusammen eine unverzweigte oder verzweigte Alkylengruppe bilden, die durch 1 oder 2 Gruppen -COOH oder -COOZ substituiert sein kann, sowie solche Verbindungen der Formel II, worin $R^1$ und $R^2$ zusammmen eine direkte Bindung bilden, wobei in allen Fällen der Rest

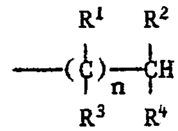

mindestens eine Gruppe -COOZ oder der Formel III oder IIIa enthält, sowie von nicht-toxischen Salzen solcher Verbindungen der Formel II, die eine oder mehrere Gruppen -COOH enthalten.

4. Verwendung von Verbindungen gemäss Anspruch 3 der Formel II, worin n = 1 ist.

5. Verwendung gemäss Anspruch 3 von Verbindungen der Formel II, worin $R^1$, $R^2$, $R^3$ und $R^4$ Wasserstoff, $C_1$-$C_4$-Alkyl, -COOH, -COOZ oder durch -COOH oder -COOZ substituiertes $C_1$-$C_4$-Alkyl darstellen.

6. Verwendung gemäss Anspruch 5 von Verbindungen der Formel II, worin $R^4$ eine Gruppe -COOH oder -COOZ oder durch -COOH oder -COOZ substituiertes Alkyl ist.

7. Verwendung gemäss Anspruch 3, wobei mindestens einer der Substituenten $R^1$, $R^2$, $R^3$ und $R^4$ -COOH oder eine durch -COOH substituierte Alkylgruppe ist und mindestens einer dieser Substituenten eine Gruppe -COOZ oder eine durch -COOZ substituierte Alkylgruppe ist.

8. Verwendung gemäss Anspruch 7, wobei sich die Gruppen -COOH und -COOZ an benachbarten C-Atomen befinden.

9. Verwendung gemäss Anspruch 3 eines Esters der Formel II, der mindestens eine Gruppe -COOZ enthält, worin Z $C_1$-$C_{18}$-Alkyl, $C_3$-$C_{12}$-Alkoxyalkyl, $C_2$-$C_6$-Hydroxyalkyl, Allyl, Cyclohexyl, Benzyl, Phenyl, Tolyl oder Naphthyl ist.

10. Antikorrosiver Anstrichstoff enthaltend ein Bindemittel und als Korrosionsinhibitor 0,1 bis 20 Gew. %, vorzugsweise 0,5 bis 5 Gew.-%, bezogen auf den Feststoffgehalt des Anstrichstoffes, eines Esters oder Anhydrides einer aliphatischen oder cycloaliphatischen Mono-, Di-, Tri- oder Tetracarbonsäure, welche im (cyclo)aliphatischen Rest durch mindestens eine Gruppe der Formel I substituiert ist,

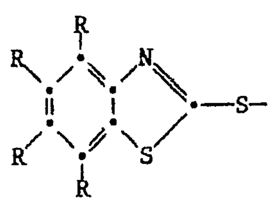
I

worin einer der Substituenten R Wasserstoff, $C_1$-$C_4$-Alkyl oder $C_{1-4}$-Alkoxy ist und die drei anderen R Wasserstoff sind, oder eines nicht-toxischen Salzes einer solchen Verbindung, die eine Gruppe COOH enthält.

11. Antikorrosiver Anstrichstoff gemäss Anspruch 10, auf nichtwässriger Basis, der als Bindemittel ein Epoxidharz, Polyurethan, Aminoplastharz, Acrylharz, Polyester, Alkydharz, Phenolharz, Polyvinylbutyral, Polyvinylacetat, Polyvinylchlorid, Chlorkautschuk, Styrol-Copolymerisat, einen Celluloseester oder ein trocknendes Oel oder eine Mischung solcher Harze enthält.

12. Antikorrosiver Anstrich gemäss Anspruch 10, auf wässriger Basis, der als Bindemittel ein Alkydharz, einen Polyester, ein Acrylharz, Polyurethan, Epoxidharz, Phenolharz, Aminoplastharz oder ein Gemisch

solcher Harze oder ein Homo- oder Copolymerisat von Vinylethern, Vinylestern, Styrol, Vinylidenchlorid oder Vinylchlorid enthält.

**13.** Antikorrosiver Anstrichstoff gemäss Anspruch 10, der einen basischen Füllstoff oder ein basisches Pigment enthält.

**14.** Antikorrosiver Anstrichstoff gmeäss Anspruch 10, der ausser dem in Anspruch 11 definierten Korrosionsinhibitor noch einen oder mehrere andere Korrosionsinhibitoren enthält, welche organische, metallorganische oder anorganische Verbindungen sein können.

**15.** Verwendung von antikorrosiven Anstrichstoffen des Anspruches 10 als Primer auf metallischen Substraten.

**16.** Verwendung gemäss Anspruch 15 als Elektrotauchlack für die kathodische Elektroabscheidung.

**17.** Antikorrosives wässriges System, das kein Anstrichstoff ist, enthaltend als Korrosionsinhibitor 0,1 ppm bis 5 Gew.-%, vorzugsweise 1 ppm bis 500 ppm eines Esters oder Anhydrides einer aliphatischen oder cycloaliphatischen Mono-, Di-, Tri- oder Tetracarbonsäure, welchhe im (cyclo)aliphatischen Rest mindestens durch eine Gruppe der Formel I substituiert ist,

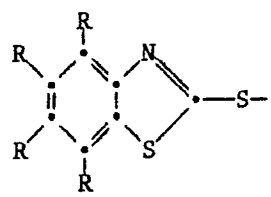

I

worin einer der Substituenten R Wasserstoff, $C_1$-$C_4$-Alkyl oder $C_1$-$C_4$-Alkoxy ist und die drei anderen R Wasserstoff sind, oder eines nicht-toxischen Salzes einer solchen Verbindung, die eine Gruppe -COOH enthält.

**18.** Antikorrosives wässriges System gemäss Anspruch 17, das ein Kühlwassersystem, eine Klimaanlage, ein Dampfgenerator, ein Heizwassersystem, eine Hydraulikflüssigkeit, ein Gefrierschutzmittel oder eine Metallbearbeitungs-Flüssigkeit ist.

**19.** Verbindungen der Formel II,

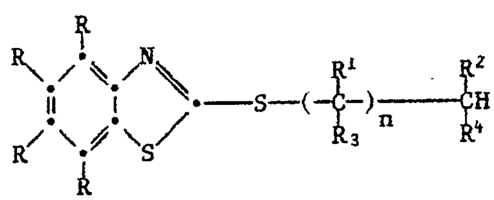

II

worin R die in Anspruch 1 gegebene Bedeutung hat, $R^1$, $R^2$, $R^3$, $R^4$ und n die in Anspruch 5 gegebene Bedeutung haben und der Rest

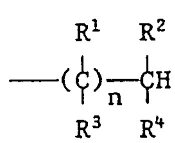

eine, zwei oder drei Gruppen -COOZ und mindestens eine Gruppe -COOH enthält.

20. Verbindungen der Formel II, worin R die in Anspruch 1 gegebene Bedeutung hat, n, $R^1$, $R^2$, $R^3$ und $R^4$ die in Anspruch 5 gegebene Bedeutung haben und der Rest

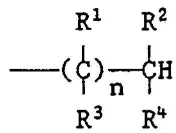

eine oder zwei Gruppen der Formel III oder IIIa enthält.

Patentansprüche für folgenden Vertragsstaat: AT

1. Verwendung von Estern oder Anhydriden aliphatischer oder cycloaliphatischer Mono-, Di-, Tri- oder Tetracarbonsäuren, die im (cyclo-)aliphatischen Rest durch eine oder mehrere der Gruppen der Formel I substituiert sind,

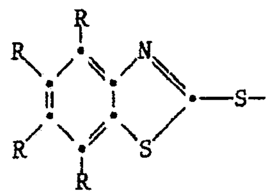

I

worin einer der Substituenten R Wasserstoff, $C_1$-$C_4$-Alkyl oder $C_1$-$C_4$-Alkoxy ist und die drei anderen R Wasserstoff sind, oder von nicht-toxischen Salzen solcher Verbindungen als Korrosionsinhibitoren.

2. Verwendung gemäss Anspruch 1 von Estern und Anhydriden, die die Gruppe I enthalten, worin alle R Wasserstoff sind.

3. Verwendung gemäss Anspruch 1 von Estern oder Anhydriden der Formel II,

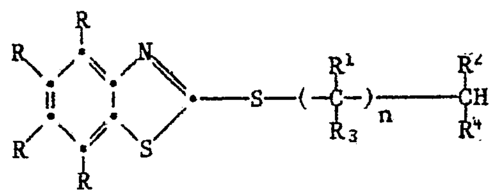

II

worin R die in Anspruch 1 gegebene Bedeutung hat, n Null oder 1 ist und
$R^1$, $R^2$, $R^3$, $R^4$ unabhängig voneinander Wasserstoff, $C_1$-$C_{18}$-Alkyl, $C_1$-$C_4$-Hydroxyalkyl, $C_1$-$C_4$-Halogenalkyl, $C_2$-$C_{10}$-Alkoxyalkyl, unsubstituiertes oder im Phenylkern durch Halogen oder Hydroxy substituiertes Phenyl oder Phenylalkyl, eine Gruppe -COOH oder -COOZ oder durch 1, 2 oder 3 Gruppen -COOH oder -COOZ substituiertes $C_2$-$C_{12}$-Alkyl bedeuten, worin Z $C_1$-$C_{18}$-Alkyl bedeutet, das durch ein oder mehrere O- oder S-Atome oder $NR^{\circ}$ -Gruppen unterbrochen sein kann (worin $R^{\circ}$ $C_1$-$C_{18}$-Alkyl, $C_5$-$C_8$-Cycloalkyl, Phenyl, Naphthyl, $C_7$-$C_9$-Phenylalkyl oder $C_7$-$C_{18}$-Alkylphenyl bedeutet) oder durch -SH. $-COOR^{\circ}$, $-CONH_2$, -CN oder Halogen substituiert sein kann, oder Z $C_2$-$C_{10}$-Hydroxyalkyl bedeutet (das durch eine oder mehrere $NR^{\circ}$-Gruppen oder O-Atome unterbrochen sein kann), oder $C_2$-$C_{18}$-Alkenyl, $C_3$-$C_{12}$-Cycloalkyl (das durch $C_1$-$C_4$-Alkyl, -OH, -SH, $-COOR^{\circ}$, $-CONH_2$, -CN oder Halogen substituiert sein kann), $C_7C_9$-Phenylalkyl, $C_7$-$C_{18}$-Alkylphenyl oder $C_6$-$C_{10}$-Aryl (das durch $C_1$-$C_{12}$-Alkoxy, $C_1$-$C_{12}$-Alkylthio, -COOH, -OH, $-NO_2$ oder Halogen substituiert sein kann) bedeutet, sowie solche Verbindungen der Formel II, worin im Rest

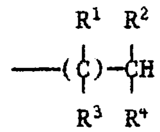

zwei Gruppen -COOH zu einer Anhydridgruppe der Formel III oder IIIa cyclisiert sind,

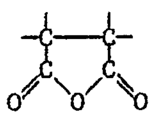 III 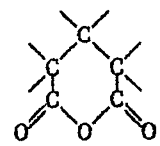 IIIa

sowie solche Verbindungen der Formel II, worin $R^1$ und $R^2$ oder $R^1$ und $R^3$ zusammen eine unverzweigte oder verzweigte Alkylengruppe bilden, die durch 1 oder 2 Gruppen -COOH oder -COOZ substituiert sein kann, sowie solche Verbindungen der Formel II, worin $R^1$ und $R^2$ zusammmen eine direkte Bindung bilden, wobei in allen Fällen der Rest

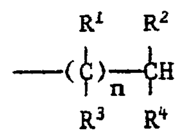

mindestens eine Gruppe -COOZ oder der Formel III oder IIIa enthält, sowie von nicht-toxischen Salzen solcher Verbindungen der Formel II, die eine oder mehrere Gruppen -COOH enthalten.

4. Verwendung von Verbindungen gemäss Anspruch 3 der Formel II, worin n = 1 ist.

5. Verwendung gemäss Anspruch 3 von Verbindungen der Formel II, worin $R^1$, $R^2$, $R^3$ und $R^4$ Wasserstoff, $C_1$-$C_4$-Alkyl, -COOH, -COOZ oder durch -COOH oder -COOZ substituiertes $C_1$-$C_4$-Alkyl darstellen.

6. Verwendung gemäss Anspruch 5 von Verbindungen der Formel II, worin $R^4$ eine Gruppe -COOH oder -COOZ oder durch -COOH oder -COOZ substituiertes Alkyl ist.

7. Verwendung gemäss Anspruch 3, wobei mindestens einer der Substituenten $R^1$, $R^2$, $R^3$ und $R^4$ -COOH oder eine durch -COOH substituierte Alkylgruppe ist und mindestens einer dieser Substituenten eine Gruppe -COOZ oder eine durch -COOZ substituierte Alkylgruppe ist.

8. Verwendung gemäss Anspruch 7, wobei sich die Gruppen -COOH und -COOZ an benachbarten C-Atomen befinden.

9. Verwendung gemäss Anspruch 3 eines Esters der Formel II, der mindestens eine Gruppe -COOZ enthält, worin Z $C_1$-$C_{18}$-Alkyl, $C_3$-$C_{12}$-Alkoxyalkyl, $C_2$-$C_6$-Hydroxyalkyl, Allyl, Cyclohexyl, Benzyl, Phenyl, Tolyl oder Naphthyl ist.

10. Antikorrosiver Anstrichstoff enthaltend ein Bindemittel und als Korrosionsinhibitor 0,1 bis 20 Gew. %, vorzugsweise 0,5 bis 5 Gew.-%, bezogen auf den Feststoffgehalt des Anstrichstoffes, eines Esters oder Anhydrides einer aliphatischen oder cycloaliphatischen Mono-, Di-, Tri- oder Tetracarbonsäure, welche im (cyclo)aliphatischen Rest durch mindestens eine Gruppe der Formel I substituiert ist,

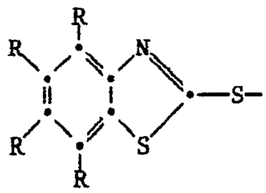 I

worin einer der Substituenten R Wasserstoff, $C_1$-$C_4$-Alkyl oder $C_{1-4}$-Alkoxy ist und die drei anderen R Wasserstoff sind, oder eines nicht-toxischen Salzes einer solchen Verbindung, die eine Gruppe COOH enthält

11. Antikorrosiver Anstrichstoff gemäss Anspruch 10, auf nichtwässriger Basis, der als Bindemittel ein Epoxidharz, Polyurethan, Aminoplastharz, Acrylharz, Polyester, Alkydharz, Phenolharz, Polyvinylbutyral, Polyvinylacetat, Polyvinylchlorid, Chlorkautschuk, Styrol-Copolymerisat, einen Celluloseester oder ein trocknendes Oel oder eine Mischung solcher Harze enthält.

12. Antikorrosiver Anstrich gemäss Anspruch 10, auf wässriger Basis, der als Bindemittel ein Alkydharz, einen Polyester, ein Acrylharz, Polyurethan, Epoxidharz, Phenolharz, Aminoplastharz oder ein Gemisch solcher Harze oder ein Homo- oder Copolymerisat von Vinylethern, Vinylestern, Styrol, Vinylidenchlorid oder Vinylchlorid enthält.

13. Antikorrosiver Anstrichstoff gemäss Anspruch 10, der einen basischen Füllstoff oder ein basisches Pigment enthält.

14. Antikorrosiver Anstrichstoff gmeäss Anspruch 10, der ausser dem in Anspruch 11 definierten Korrosionsinhibitor noch einen oder mehrere andere Korrosionsinhibitoren enthält, welche organische, metallorganische oder anorganische Verbindungen sein können.

15. Verwendung von antikorrosiven Anstrichstoffen des Anspruches 10 als Primer auf metallischen Substraten.

16. Verwendung gemäss Anspruch 15 als Elektrotauchlack für die kathodische Elektroabscheidung.

17. Antikorrosives wässriges System, das kein Anstrichstoff ist, enthaltend als Korrosionsinhibitor 0,1 ppm bis 5 Gew.-%, vorzugsweise 1 ppm bis 500 ppm eines Esters oder Anhydrides einer aliphatischen oder cycloaliphatischen Mono-, Di-, Tri- oder Tetracarbonsäure, welchhe im (cyclo)aliphatischen Rest mindestens durch eine Gruppe der Formel I substituiert ist,

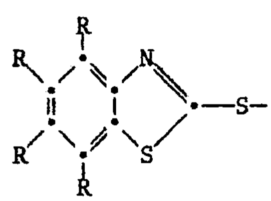 I

worin einer der Substituenten R Wasserstoff, $C_1$-$C_4$-Alkyl oder $C_1$-$C_4$-Alkoxy ist und die drei anderen R Wasserstoff sind, oder eines nicht-toxischen Salzes einer solchen Verbindung, die eine Gruppe -COOH enthält.

18. Antikorrosives wässriges System gemäss Anspruch 17, das ein Kühlwassersystem, eine Klimaanlage, ein Dampfgenerator, ein Heizwassersystem, eine Hydraulikflüssigkeit, ein Gefrierschutzmittel oder eine Metallbearbeitungs-Flüssigkeit ist.

## Claims

1. Use of an ester or anhydride of an aliphatic or cycloaliphatic mono-, di-, tri- or tetracarboxylic acid which is substituted in the (cyclo)aliphatic radical by one or more groups of the formula I

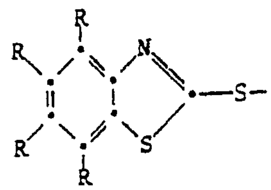

I

in which one of the substituents R is hydrogen, $C_1$-$C_4$alkyl or $C_1$-$C_4$alkoxy and the three other substituents R are hydrogen, or of non-toxic salts of such compounds, as corrosion inhibitor.

2. Use according to claim 1 of an ester or anhydride which contains the group I in which all substituents R are hydrogen.

3. Use according to claim 1 of an ester or anhydride of the formula II

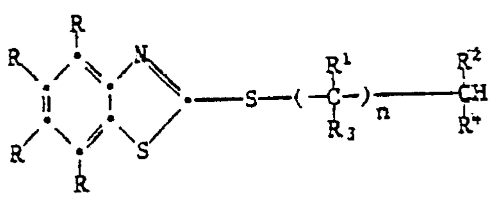

II

in which R is as defined in claim 1, n is zero or 1 and $R^1$, $R^2$, $R^3$, $R^4$ independently of one another are hydrogen, $C_1$-$C_{18}$alkyl, $C_1$-$C_4$hydroxyalkyl, $C_1$-$C_4$haloalkyl, $C_2$-$C_{10}$alkoxyalkyl, or phenyl or phenylalkyl each of which is unsubstituted or substituted in the phenyl nucleus by halogen or hydroxyl, or a -COOH or -COOZ group, or $C_2$-$C_{12}$alkyl substituted by 1, 2 or 3 -COOH or -COOZ groups in which Z is $C_1$-$C_{18}$alkyl which may be interrupted by one or more O or S atoms or $NR^\circ$ groups (in which $R^\circ$ is $C_1$-$C_{18}$alkyl. $C_5$-$C_8$cycloalkyl, phenyl, naphthyl, $C_7$-$C_9$-phenylalkyl or $C_7$-$C_{18}$alkylphenyl) or may be substituted by -SH, $-COOR^\circ$, $-CONH_2$, -CN or halogen, or Z is $C_2$-$C_{10}$hydroxyalkyl (which may be interrupted by one or more $NR^\circ$ groups or O atoms), or is $C_2$-$C_{18}$alkenyl, $C_3$-$C_{12}$cycloalkyl (which may be substituted by $C_1$-$C_4$alkyl, -OH, -SH, $-COOR^\circ$, $-CONH_2$, -CN or halogen), $C_7$-$C_9$phenylalkyl, $C_7$-$C_{18}$alkylphenyl or $C_6$-$C_{10}$aryl (which may be substituted by $C_1$-$C_{12}$alkoxy, $C_1$-$C_{12}$alkylthio, -COON, -OH, $-NO_2$, or halogen), and also those compounds of the formula II in which in the radical

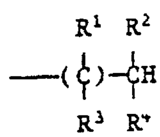

two -COOH groups are cyclised to an anhydride group of the formula III or IIIa

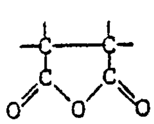

III

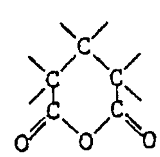

IIIa

and also those compounds of the formula II, in which $R^1$ and $R^2$ or $R^1$ and $R^3$ together form an unbranched or branched alkylene group which may be substituted by 1 or 2 -COOH or -COOZ groups, and also those compounds of the formula II in which $R^1$ and $R^2$ together form a direct bond, where the

radical

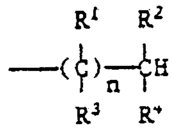

contains at least one -COOZ group or one group of the formula III or IIIa in every case, or of a non-toxic salt of a compound of the formula II which contains one or more -COOH groups.

**4.** Use of a compound according to claim 3 of the formula II in which n is 1.

**5.** Use according to claim 3 of a compound of the formula II in which $R^1$, $R^2$, $R^3$ and $R^4$ are hydrogen, $C_1$-$C_4$alkyl, -COOH, -COOZ or $C_1$-$C_4$alkyl substituted by -COOH or -COOZ.

**6.** Use according to claim 5 of a compound of the formula II in which $R^4$ is a -COOH or -COOZ group or alkyl substituted by -COOH or -COOZ.

**7.** Use according to claim 3, where at least one of the substituents $R^1$, $R^2$, $R^3$ and $R^4$ is -COOH or an alkyl group substituted by -COOH and at least one of these substituents is a -COOZ group or an alkyl group substituted by -COOZ.

**8.** Use according to claim 7, where the -COOH and -COOZ groups are situated on adjacent carbon atoms.

**9.** Use according to claim 3 of an ester of the formula II which contains at least one -COOZ group in which Z is $C_1$-$C_{18}$alkyl, $C_3$-$C_{12}$alkoxyalkyl, $C_2$-$C_6$hydroxyalkyl, allyl, cyclohexyl, benzyl, phenyl, tolyl or naphthyl.

**10.** Anticorrosive coating composition containing a binder and as corrosion inhibitor 0.1 to 20 % by weight, preferably 0.5 to 5 % by weight, based on the solids content of the coating composition, of an ester or anhydride of an aliphatic or cycloaliphatic mono-, di-, tri- or tetracarboxylic acid which is substituted in the (cyclo)aliphatic radical by at least one group of the formula I

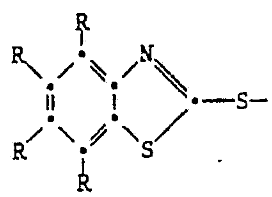

I

in which one of the substituents R is hydrogen, $C_1$-$C_4$alkyl or $C_1$-$C_4$alkoxy and the three other substituents R are hydrogen, or of a non-toxic salt of such a compound which contains a -COOH group.

**11.** Anticorrosive coating composition according to claim 10 on a non-aqueous basis, which contains as binder an epoxy resin, polyurethane, amino resin, acrylic resin, polyester, alkyd resin, phenolic resin, polyvinyl butyral, polyvinyl acetate, polyvinyl chloride, chlorinated rubber, styrene copolymer, a cellulose ester or a drying oil or a mixture of such resins.

**12.** Anticorrosive coating according to claim 10 on an aqueous basis, which contains as binder an alkyd resin, a polyester, an acrylic resin, polyurethane, epoxy resin, phenolic resin, amino resin or a mixture of such resins, or a homopolymer or copolymer of vinyl ethers, vinyl esters, styrene, vinylidene chloride or vinyl chloride.

**13.** Anticorrosive coating composition according to claim 10 which contains a basic filler or a basic pigment.

14. Anticorrosive coating composition according to claim 10 which, apart from the corrosion inhibitor defined in claim 11, also contains one or more other corrosion inhibitors which may be organic, organometallic or inorganic compounds.

15. Use of an anticorrosive coating composition of claim 10 as primer on metallic substrates.

16. Use according to claim 15 as an electrodeposition coating for cathodic electrodeposition.

17. Anticorrosive aqueous system, which is not a coating composition, containing as corrosion inhibitor 0.1 ppm to 5% by weight, preferably 1 ppm to 500 ppm, of an ester or anhydride of an aliphatic or cycloaliphatic mono-, di-, tri- or tetracarboxylic acid which is substituted in the (cyclo)aliphatic radical at least by one group of the formula I

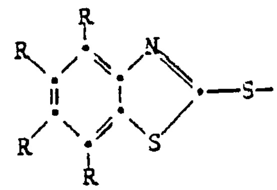
I

in which one of the substituents R is hydrogen, $C_1$-$C_4$alkyl or $C_1$-$C_4$alkoxy and the three other substituents R are hydrogen, or of a non-toxic salt of such a compound which contains a -COOH group.

18. Anticorrosive aqueous system according to claim 17, which is a cooling water system, an air-conditioning system, a steam generator, a hot water system, a hydraulic fluid, an antifreeze composition or a metal-working fluid.

19. A compound of the formula II

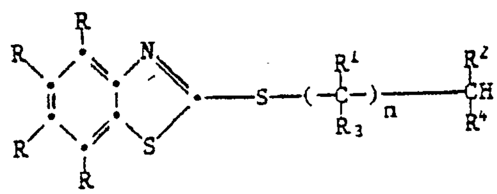
II

in which R is as defined in claim 1, $R^1$, $R^2$, $R^3$, $R^4$ and n are as defined in claim 5 and the radical

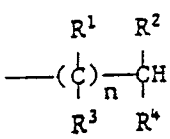

contains one, two or three -COOZ groups and at least one -COOH group.

20. A compound of the formula II in which R is as defined in claim 1, n, $R^1$, $R^2$, $R^3$, and $R^4$ are as defined in claim 5 and the radical

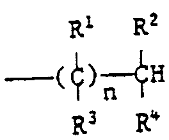

contains one or two groups of the formula III or IIIa.

Claims for the following Contracting State: AT

1. Use of an ester or anhydride of an aliphatic or cycloaliphatic mono-, di-, tri- or tetracarboxylic acid which is substituted in the (cyclo)aliphatic radical by one or more groups of the formula I

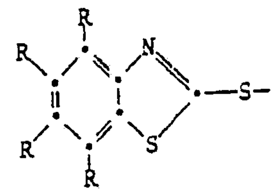

I

in which one of the substituents R is hydrogen, $C_1$-$C_4$alkyl or $C_1$-$C_4$alkoxy and the three other substituents R are hydrogen, or of non-toxic salts of such compounds, as corrosion inhibitor.

2. Use according to claim 1 of an ester or anhydride which contains the group I in which all substituents R are hydrogen.

3. Use according to claim 1 of an ester or anhydride of the formula II

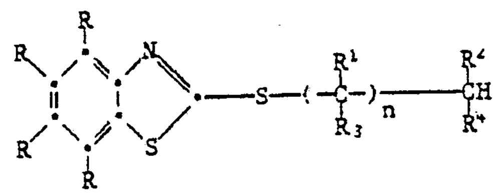

II

in which R is as defined in claim 1, n is zero or 1 and $R^1$, $R^2$, $R^3$, $R^4$ independently of one another are hydrogen, $C_1$-$C_{18}$alkyl, $C_1$-$C_4$hydroxyalkyl, $C_1$-$C_4$haloalkyl, $C_2$-$C_{10}$alkoxyalkyl, or phenyl or phenylalkyl each of which is unsubstituted or substituted in the phenyl nucleus by halogen or hydroxyl, or a -COOH or -COOZ group, or $C_2$-$C_{12}$alkyl substituted by 1, 2 or 3 -COOH or -COOZ groups in which Z is $C_1$-$C_{18}$alkyl which may be interrupted by one or more O or S atoms or $NR^\circ$ groups (in which $R^\circ$ is $C_1$-$C_{18}$alkyl, $C_5$-$C_8$cycloalkyl, phenyl, naphthyl, $C_7$-$C_9$-phenylalkyl or $C_7$-$C_{18}$alkylphenyl) or may be substituted by -SH, $-COOR^\circ$, $-CONH_2$, -CN or halogen, or Z is $C_2$-$C_{10}$hydroxyalkyl (which may be interrupted by one or more $NR^\circ$ groups or O atoms), or is $C_2$-$C_{18}$alkenyl, $C_3$-$C_{12}$cycloalkyl (which may be substituted by $C_1$-$C_4$alkyl, -OH, -SH, $-COOR^\circ$, $-CONH_2$, -CN or halogen), $C_7$-$C_9$phenylalkyl, $C_7$-$C_{18}$alkylphenyl or $C_6$-$C_{10}$aryl (which may be substituted by $C_1$-$C_{12}$alkoxy, $C_1$-$C_{12}$alkylthio, -COOH, -OH, $-NO_2$, or halogen), and also those compounds of the formula II in which in the radical

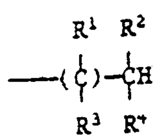

two -COOH groups are cyclised to an anhydride group of the formula III or IIIa

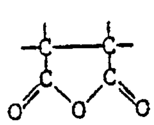

III

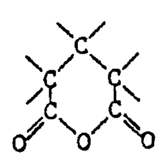

IIIa

and also those compounds of the formula II, in which $R^1$ and $R^2$ or $R^1$ and $R^3$ together form an unbranched or branched alkylene group which may be substituted by 1 or 2 -COOH or -COOZ groups,

and also those compounds of the formula II in which $R^1$ and $R^2$ together form a direct bond, where the radical

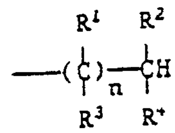

contains at least one -COOZ group or one group of the formula III or IIIa in every case, or of a non-toxic salt of a compound of the formula II which contains one or more -COOH groups.

4. Use of a compound according to olaim 3 of the formula II in which n is 1.

5. Use according to claim 3 of a compound of the formula II in which $R^1$, $R^2$, $R^3$ and $R^4$ are hydrogen, $C_1$-$C_4$alkyl, -COON, -COOZ or $C_1$-$C_4$alkyl substituted by -COOH or -COOZ.

6. Use according to claim 5 of a compound of the formula II in which $R^4$ is a -COOH or -COOZ group or alkyl substituted by -COOH or -COOZ.

7. Use according to claim 3, where at least one of the substituents $R^1$, $R^2$, $R^3$ and $R^4$ is -COOH or an alkyl group substituted by -COOH and at least one of these substituents is a -COOZ group or an alkyl group substituted by -COOZ.

8. Use according to claim 7, where the -COOH and -COOZ groups are situated on adjacent carbon atoms.

9. Use according to claim 3 of an ester of the formula II which contains at least one -COOZ group in which Z is $C_1$-$C_{18}$alkyl, $C_3$-$C_{12}$alkoxyalkyl, $C_2$-$C_6$hydroxyalkyl, allyl, cyclohexyl, benzyl, phenyl, tolyl or naphthyl.

10. Anticorrosive coating composition containing a binder and as corrosion inhibitor 0.1 to 20 % by weight, preferably 0.5 to 5 % by weight, based on the solids content of the coating composition, of an ester or anhydride of an aliphatic or cycloaliphatic mono-, di-, tri- or tetracarboxylic acid which is substituted in the (cyclo)aliphatic radical by at least one group of the formula I

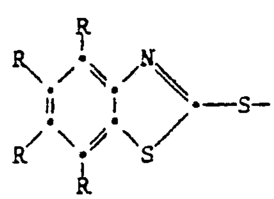

I

in which one of the substituents R is hydrogen. $C_1$-$C_4$alkyl or $C_1$-$C_4$alkoxy and the three other substituents R are hydrogen, or of a non-toxic salt of such a compound which contains a -COOH group.

11. Anticorrosive coating composition according to claim 10 on a non-aqueous basis, which contains as binder an epoxy resin, polyurethane, amino resin, acrylic resin, polyester, alkyd resin, phenolic resin, polyvinyl butyral, polyvinyl acetate, polyvinyl chloride, chlorinated rubber, styrene copolymer, a cellulose ester or a drying oil or a mixture of such resins.

12. Anticorrosive coating according to claim 10 on an aqueous basis, which contains as binder an alkyd resin, a polyester, an acrylic resin, polyurethane, epoxy resin, phenolic resin, amino resin or a mixture of such resins, or a homopolymer or copolymer of vinyl ethers, vinyl esters, styrene, vinylidene chloride or vinyl chloride.

13. Anticorrosive coating composition according to claim 10 which contains a basic filler or a basic pigment.

14. Anticorrosive coating composition according to claim 10 which, apart from the corrosion inhibitor defined in claim 11, also contains one or more other corrosion inhibitors which may be organic, organometallic or inorganic compounds.

15. Use of an anticorrosive coating composition of claim 10 as primer on metallic substrates.

16. Use according to claim 15 as cathodic electrodeposition coating for cathodic electrodeposition.

17. Anticorrosive aqueous system, which is not a coating composition, containing as corrosion inhibitor 0.1 ppm to 5% by weight, preferably 1 ppm to 500 ppm, of an ester or anhydride of an aliphatic or cycloaliphatic mono-, di-, tri- or tetracarboxylic acid which is substituted in the (cyclo)aliphatic radical at least by one group of the formula I

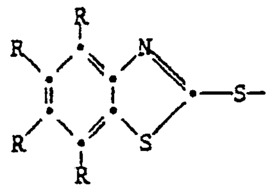

I

in which one of the substituents R is hydrogen, $C_1$-$C_4$alkyl or $C_1$-$C_4$alkoxy and the three other substituents R are hydrogen, or of a non-toxic salt of such a compound which contains a -COOH group.

18. Anticorrosive aqueous system according to claim 17, which is a cooling water system, an air-conditioning system, a steam generator, a hot water system, a hydraulic fluid, an antifreeze composition or a metal-working fluid.

## Revendications

1. Application d'esters ou d'anhydrides d'acides mono-, di-, tri- ou tétra-carboxyliques aliphatiques ou cycloaliphatiques qui portent, sur le radical aliphatique ou cycloaliphatique, un ou plusieurs radicaux répondant à la formule I :

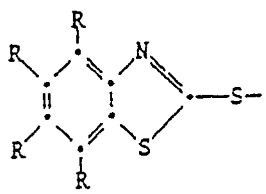

I

dans laquelle un des symboles R représente l'hydrogène, un alkyle en $C_1$-$C_4$ ou un alcoxy en $C_1$-$C_4$ et les trois autres symboles R représentent chacun l'hydrogène,
ou de sels non toxiques de ces composés, comme inhibiteurs de corrosion.

2. Application selon la revendication 1 d'esters et d'anhydrides qui contiennent un radical (I) dans lequel tous les R représentent l'hydrogène.

3. Application selon la revendication 1 :
   - d'esters ou d'anhydrides répondant à la formule II :

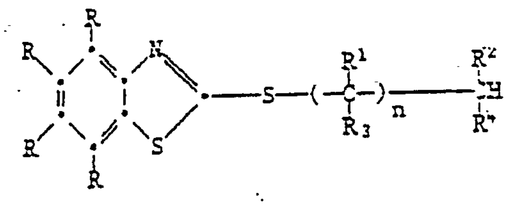 II

dans laquelle les R ont les significations qui leur ont été données à la revendication 1, n est égal à 0 ou à 1, et $R^1$, $R^2$, $R^3$ et $R^4$ représentent chacun, indépendamment les uns des autres, l'hydrogène, un alkyle en $C_1$-$C_{18}$, un hydroxyalkyle en $C_1$-$C_4$, un halogéno-alkyle en $C_1$-$C_4$, un alcoxy-alkyle en $C_2$-$C_{10}$, un radical phényle ou phénylalkyle non substitué ou porteur, sur son phényle, d'un halogène ou d'un hydroxy, un radical -COOH ou -COOZ ou un alkyle en $C_2$-$C_{12}$ porteur d'un, de deux ou de trois radicaux -COOH ou -COOZ, le symbole Z représentant un alkyle en $C_1$-$C_{18}$ qui peut être interrompu par un ou plusieurs atomes O ou S ou radicaux $NR^{\circ}$ (dans lesquels $R^{\circ}$ représente un alkyle en $C_1$-$C_{18}$, un cycloalkyle en $C_5$-$C_8$, un phényle, un naphtyle, un phénylalkyle en $C_7$-$C_9$ ou un alkylphényle en $C_7$-$C_{18}$) ou qui peut porter un radical -SH, $-COOR^{\circ}$, $-CONH_2$ ou -CN ou un halogène, ou Z représentant un hydroxy-alkyle en $C_2$-$C_{10}$ (qui peut être interrompu par un ou plusieurs radicaux $NR^{\circ}$ ou atomes O), ou un alcényle en $C_2$-$C_{18}$, un cycloalkyle en $C_3$-$C_{12}$ (qui peut porter un alkyle en $C_1$-$C_4$, un -OH, un -SH, un $-COOR^{\circ}$, un $-CONH_2$, un -CN ou un halogène), un phénylalkyle en $C_7$-$C_9$, un alkylphényle en $C_7$-$C_{18}$ ou un aryle en $C_6$-$C_{10}$ (qui peut porter un alcoxy en $C_1$-$C_{12}$, un alkylthio en $C_1$-$C_{12}$, un -COOH, un -OH, un $-NO_2$ ou un halogène),

- de composés de formule II dans lesquels deux radicaux -COOH contenus dans le radical :

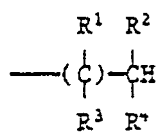

sont cyclisés et forment un radical d'anhydride de formule III ou IIIa,

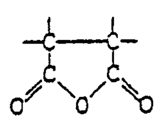 III 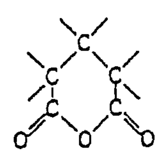 IIIa,

- de composés de formule II dans lesquels $R^1$ et $R^2$, ou $R^1$ et $R^3$, forment ensemble un radical alkylène, ramifié ou non, qui peut porter 1 ou 2 radicaux -COOH ou -COOZ,
- de composés de formule II dans lesquels $R^1$ et $R^2$ forment ensemble une liaison directe,

avec la condition que, dans tous les cas, le radical :

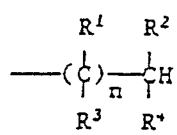

contienne au moins un radical de formule -COOZ ou de formule III ou IIIa,
et de sels non toxiques de tels composés de formule II qui contiennent un ou plusieurs radicaux -COOH.

4. Application selon la revendication 3 de composés de formule II dans lesquels n est égal à 1.

5. Application selon la revendication 3 de composés de formule II dans lesquels $R^1$, $R^2$, $R^3$ et $R^4$ représentent chacun l'hydrogène, un alkyle en $C_1$-$C_4$, un -COOH, un -COOZ ou un alkyle en $C_1$-$C_4$ qui porte un radical -COOH ou -COOZ.

6. Application selon la revendication 5 de composés de formule II dans lesquels $R^4$ représente un radical -COOH ou -COOZ ou un alkyle porteur d'un radical -COOH ou -COOZ.

7. Application selon la revendication 3 selon laquelle au moins un des symboles $R^1$, $R^2$, $R^3$ et $R^4$ représente un radical -COOH ou un alkyle porteur d'un radical -COOH, et au moins un de ces symboles représente un radical -COOZ ou un alkyle porteur d'un radical -COOZ.

8. Application selon la revendication 7 selon laquelle les radicaux -COOH et -COOZ se trouvent sur des atomes de carbone voisins.

9. Application selon la revendication 3 d'un ester de formule II qui contient au moins un radical -COOZ dans lequel Z représente un alkyle en $C_1$-$C_{18}$, un alcoxyalkyle en $C_3$-$C_{12}$, un hydroxyalkyle en $C_2$-$C_6$, un allyle, un cyclohexyle, un benzyle, un phényle, un tolyle ou un naphtyle.

10. Peinture anti-corrosion qui contient un liant et, comme inhibiteur de corrosion, de 0,1 à 20% en poids, de préférence de 0,5 à 5% en poids, par rapport à la matière solide de la peinture, d'un ester ou d'un anhydride d'un acide mono-, di-, tri- ou tétra-carboxylique aliphatique ou cycloaliphatique qui porte, sur son radical aliphatique ou cycloaliphatique, au moins un radical répondant à la formule I :.

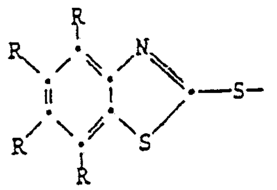

I

dans laquelle un des symboles R représente l'hydrogène, un alkyle en $C_1$-$C_4$ ou un alcoxy en $C_1$-$C_4$ et les trois autres R représentent chacun l'hydrogène,
ou d'un sel non toxique d'un tel composé qui contient un radical -COOH.

11. Peinture anti-corrosion selon la revendication 10 qui est à base non aqueuse et qui contient, comme liant, une résine époxydique, un polyuréthanne, une résine aminoplaste, une résine acrylique, un polyester, une résine alkyde, une résine phénolique, du poly-(vinylbutyral), du poly-(acétate de vinyle), du poly-(chlorure de vinyle), du caoutchouc chloré, un copolymère du styrène, un ester de la cellulose ou une huile siccative, ou un mélange de telles résines.

12. Peinture anti-corrosion selon la revendication 10 qui est à base aqueuse et qui contient, comme liant, une résine alkyde, un polyester, une résine acrylique, un polyuréthanne, une résine époxydique, une résine phénolique, une résine aminoplaste ou un mélange de telles résines, ou un homopolymère ou copolymère d'éthers vinyliques, d'esters vinyliques, du styrène, du chlorure de vinylidène ou du chlorure de vinyle.

13. Peinture anti-corrosion selon la revendication 10 qui contient une charge basique ou un pigment basique.

14. Peinture anti-corrosion selon la revendication 10 qui, en plus de l'inhibiteur de corrosion défini à la revendication 11, contient un ou plusieurs autres inhibiteurs de corrosion, lesquels peuvent être des composés organiques, organo-métalliques ou minéraux.

15. Application de peintures anti-corrosions selon la revendication 10 comme peintures de fond sur substrats métalliques.

16. Application selon la revendication 15 comme peinture de trempage électrophorétique pour l'électrodéposition cathodique.

**17.** Système aqueux anti-corrosion qui n'est pas une peinture et qui contient, comme inhibiteur de corrosion, de 0,1 ppm à 5% en poids, de préférence de 1 ppm à 500 ppm, d'un ester ou d'un anhydride d'un acide mono-, di-, tri- ou tétra-carboxylique aliphatique ou cycloaliphatique portant, sur sa partie aliphatique ou cycloaliphatique, au moins un radical répondant à la formule I :

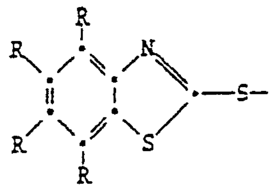

I

dans laquelle un des symboles R représente l'hydrogène, un alkyle en $C_1$-$C_4$ ou un alcoxy en $C_1$-$C_4$ et les trois autres R représentent chacun l'hydrogène,
ou d'un sel non toxique d'un tel composé qui contient un radical -COOH.

**18.** Système aqueux anti-corrosion selon la revendication 17 qui est un système d'eau de refroidissement, une installation de climatisation, un générateur de vapeur, un système d'eau de chauffage, un liquide hydraulique, un antigel ou un liquide pour l'usinage de métaux.

**19.** Composés répondant à la formule II :

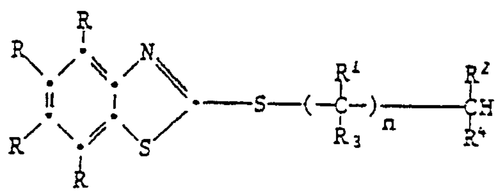

II

dans laquelle les R ont les significations qui leur ont été données à la revendication 1, $R^1$, $R^2$, $R^3$, $R^4$ et n ont les significations qui leur ont été données à la revendication 5 et le radical :

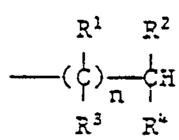

contient un, deux ou trois radicaux -COOZ et au moins un radical -COOH.

**20.** Composés de formule II dans lesquels les R ont les significations qui leur ont été données à la revendication 1, n, $R^1$, $R^2$, $R^3$ et $R^4$ ont les significations qui leur ont été données à la revendication 5 et le radical:

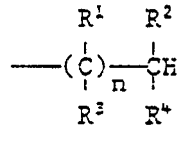

contient un ou deux radicaux de formule III ou IIIa.

Revendications pour l'Etat Contractant suivant: AT

1. Application d'esters ou d'anhydrides d'acides mono-, di-, tri- ou tétra-carboxyliques aliphatiques ou cycloaliphatiques qui portent, sur le radical aliphatique ou cycloaliphatique, un ou plusieurs radicaux répondant à la formule I :

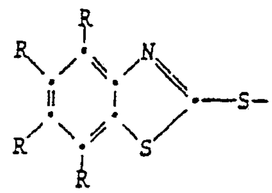

I

dans laquelle un des symboles R représente l'hydrogène, un alkyle en $C_1$-$C_4$ ou un alcoxy en $C_1$-$C_4$ et les trois autres symboles R représentent chacun l'hydrogène, ou de sels non toxiques de ces composés, comme inhibiteurs de corrosion.

2. Application selon la revendication 1 d'esters et d'anhydrides qui contiennent un radical (I) dans lequel tous les R représentent l'hydrogène.

3. Application selon la revendication 1 :
   - d'esters ou d'anhydrides répondant à la formule II :

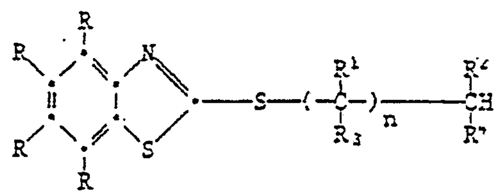

II

dans laquelle les R ont les significations qui leur ont été données à la revendication 1, n est égal à 0 ou à 1, et $R^1$, $R^2$, $R^3$ et $R^4$ représentent chacun, indépendamment les uns des autres, l'hydrogène, un alkyle en $C_1$-$C_{18}$, un hydroxyalkyle en $C_1$-$C_4$, un halogéno-alkyle en $C_1$-$C_4$, un alcoxy-alkyle en $C_2$-$C_{10}$, un radical phényle ou phénylalkyle non substitué ou porteur, sur son phényle, d'un halogène ou d'un hydroxy, un radical -COOH ou -COOZ ou un alkyle en $C_2$-$C_{12}$ porteur d'un, de deux ou de trois radicaux -COOH ou -COOZ, le symbole Z représentant un alkyle en $C_1$-$C_{18}$ qui peut être interrompu par un ou plusieurs atomes O ou S ou radicaux $NR^°$ (dans lesquels $R^°$ représente un alkyle en $C_1$-$C_{18}$, un cycloalkyle en $C_5$-$C_8$, un phényle, un naphtyle, un phénylalkyle en $C_7$-$C_9$ ou un alkylphényle en $C_7$-$C_{18}$) ou qui peut porter un radical -SH, $-COOR^°$, $-CONH_2$ ou -CN ou un halogène, ou Z représentant un hydroxy-alkyle en $C_2$-$C_{10}$ (qui peut être interrompu par un ou plusieurs radicaux $NR^°$ ou atomes O), ou un alcényle en $C_2$-$C_{18}$, un cycloalkyle en $C_3$-$C_{12}$ (qui peut porter un alkyle en $C_1$-$C_4$, un -OH, un -SH, un $-COOR^°$, un $-CONH_2$, un -CN ou un halogène), un phénylalkyle en $C_7$-$C_9$, un alkylphényle en $C_7$-$C_{18}$ ou un aryle en $C_6$-$C_{10}$ (qui peut porter un alcoxy en $C_1$-$C_{12}$, un alkylthio en $C_1$-$C_{11}$, un -COOH, un -OH, un $-NO_2$ ou un halogène),
   - de composés de formule II dans lesquels deux radicaux -COOH contenus dans le radical :

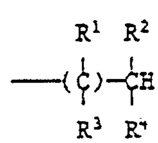

sont cyclisés et forment un radical d'anhydride de formule III ou IIIa,

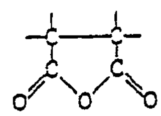 III 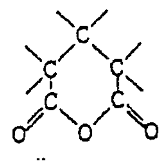 IIIa

- de composés de formule II dans lesquels $R^1$ et $R^2$, ou $R^1$ et $R^3$, forment ensemble un radical alkylène, ramifié ou non, qui peut porter 1 ou 2 radicaux -COOH ou -COOZ,
- de composés de formule II dans lesquels $R^1$ et $R^2$ forment ensemble une liaison directe,

avec la condition que, dans tous les cas, le radical :

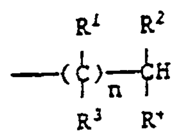

contienne au moins un radical de formule -COOZ ou de formule III ou IIIa,
et de sels non toxiques de tels composés de formule II qui contiennent un ou plusieurs radicaux -COOH.

**4.** Application selon la revendication 3 de composés de formule II dans lesquels n est égal à 1.

**5.** Application selon la revendication 3 de composés de formule II dans lesquels $R^1$, $R^2$, $R^3$ et $R^4$ représentent chacun l'hydrogène, un alkyle en $C_1$-$C_4$, un -COOH, un -COOZ ou un alkyle en $C_1$-$C_4$ qui porte un radical -COOH ou -COOZ.

**6.** Application selon la revendication 5 de composés de formule II dans lesquels $R^4$ représente un radical -COOH ou -COOZ ou un alkyle porteur d'un radical -COOH ou -COOZ.

**7.** Application selon la revendication 3 selon laquelle au moins un des symboles $R^1$, $R^2$, $R^3$ et $R^4$ représente un radical -COOH ou un alkyle porteur d'un radical -COOH, et au moins un de ces symboles représente un radical -COOZ ou un alkyle porteur d'un radical -COOZ.

**8.** Application selon la revendication 7 selon laquelle les radicaux -COOH et -COOZ se trouvent sur des atomes de carbone voisins.

**9.** Application selon la revendication 3 d'un ester de formule II qui contient au moins un radical -COOZ dans lequel Z représente un alkyle en $C_1$-$C_{18}$; un alcoxyalkyle en $C_2$-$C_{12}$, un hydroxyalkyle en $C_2$-$C_6$, un allyle, un cyclohexyle, un benzyle, un phényle, un tolyle ou un naphtyle.

**10.** Peinture anti-corrosion qui contient un liant et, comme inhibiteur de corrosion, de 0,1 à 20% en poids, de préférence de 0,5 à 5% en poids, par rapport à la matière solide de la peinture, d'un ester ou d'un anhydride d'un acide mono-, di-, tri- ou tétra-carboxylique aliphatique ou cycloaliphatique qui porte, sur son radical aliphatique ou cycloaliphatique, au moins un radical répondant à la formule I :.

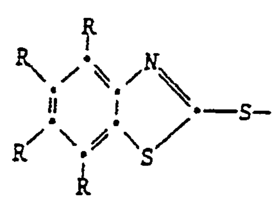 I

dans laquelle un des symboles R représente l'hydrogène, un alkyle en $C_1$-$C_4$ ou un alcoxy en $C_1$-$C_4$ et les trois autres R représentent chacun l'hydrogène,
ou d'un sel non toxique d'un tel composé qui contient un radical -COOH.

**11.** Peinture anti-corrosion selon la revendication 10 qui est à base non aqueuse et qui contient, comme liant, une résine époxydique, un polyuréthanne, une résine aminoplaste, une résine acrylique, un polyester, une résine alkyde, une résine phénolique, du poly-(vinylbutyral), du poly-(acétate de vinyle), du poly-(chlorure de vinyle), du caoutchouc chloré, un copolymère du styrène, un ester de la cellulose ou une huile siccative, ou un mélange de telles résines.

**12.** Peinture anti-corrosion selon la revendication 10 qui est à base aqueuse et qui contient, comme liant, une résine alkyde, un polyester, une résine acrylique, un polyuréthanne, une résine époxydique, une résine phénolique, une résine aminoplaste ou un mélange de telles résines, ou un homopolymère ou copolymère d'éthers vinyliques, d'esters vinyliques, du styrène, du chlorure de vinylidène ou du chlorure de vinyle.

**13.** Peinture anti-corrosion selon la revendication 10 qui contient une charge basique ou un pigment basique.

**14.** Peinture anti-corrosion selon la revendication 10 qui, en plus de l'inhibiteur de corrosion défini à la revendication 11, contient un ou plusieurs autres inhibiteurs de corrosion, lesquels peuvent être des composés organiques, organo-métalliques ou minéraux.

**15.** Application de peintures anti-corrosions selon la revendication 10 comme peintures de fond sur substrats métalliques.

**16.** Application selon la revendication 15 comme peinture de trempage électrophorétique pour l'électrodéposition cathodique.

**17.** Système aqueux anti-corrosion qui n'est pas une peinture et qui contient, comme inhibiteur de corrosion, de 0,1 ppm à 5% en poids, de préférence de 1 ppm à 500 ppm, d'un ester ou d'un anhydride d'un acide mono-, di-, tri- ou tétra-carboxylique aliphatique ou cycloaliphatique portant, sur sa partie aliphatique ou cycloaliphatique, au moins un radical répondant à la formule I :

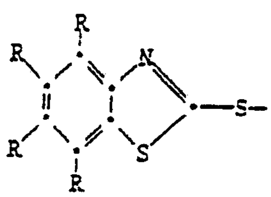

I

dans laquelle un des symboles R représente l'hydrogène, un alkyle en $C_1$-$C_4$ ou un alcoxy en $C_1$-$C_4$ et les trois autres R représentent chacun l'hydrogène,
ou d'un sel non toxique d'un tel composé qui contient un radical -COOH.

**18.** Système aqueux anti-corrosion selon la revendication 17 qui est un système d'eau de refroidissement, une installation de climatisation, un générateur de vapeur, un système d'eau de chauffage, un liquide hydraulique, un antigel ou un liquide pour l'usinage de métaux.